(19) Europäisches Patentamt
European Patent Office
Office européen des brevets

(11) EP 1 934 188 B1

(12) **EUROPÄISCHE PATENTSCHRIFT**

(45) Veröffentlichungstag und Bekanntmachung des Hinweises auf die Patenterteilung:
**27.01.2010 Patentblatt 2010/04**

(21) Anmeldenummer: **06805782.7**

(22) Anmeldetag: **20.09.2006**

(51) Int Cl.:
*C07D 249/20* (2006.01) *C07D 319/06* (2006.01)
*C07C 49/248* (2006.01) *C07C 49/252* (2006.01)

(86) Internationale Anmeldenummer:
**PCT/EP2006/009151**

(87) Internationale Veröffentlichungsnummer:
**WO 2007/039110 (12.04.2007 Gazette 2007/15)**

(54) **ALPHA, ALPHA‘-DIHYDROXYKETONDERIVATE UND DEREN VERWENDUNG ALS UV-FILTER**

ALPHA, ALPHA‘-DIHYDROXY KETONE DERIVATIVES AND USE THEREOF AS UV FILTER

DERIVES DE ALPHA, ALPHA‘-DIHYDROXYCETONE ET LEUR UTILISATION EN TANT QUE FILTRE UV

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **05.10.2005 DE 102005047647**

(43) Veröffentlichungstag der Anmeldung:
**25.06.2008 Patentblatt 2008/26**

(60) Teilanmeldung:
**09009263.6 / 2 108 646**

(73) Patentinhaber: **Merck Patent GmbH**
**64293 Darmstadt (DE)**

(72) Erfinder:
- **RUDOLPH, Thomas**
  **64291 Darmstadt (DE)**
- **BUCHHOLZ, Herwig**
  **60599 Frankfurt (DE)**

(56) Entgegenhaltungen:
**EP-A- 1 481 663** **WO-A-93/16026**
**WO-A-95/16049** **WO-A-2005/004826**
**DE-A1- 10 341 654** **US-B1- 6 288 066**

- **COLETTE DEMUYNCK, JEAN BOLTE, LAURENCE HECQUET, VALERIE DALMAS: "Enzyme-catalyzed synthesis of carbohydrates: synthetic potential of transketolase" TETRAHEDRON LETTERS, Bd. 32, Nr. 38, 1991, Seiten 5085-5088, XP002423414**
- **KAZUTSUGU MATSUMOTO, MASAYUKI SHIMAGAKI, TADASHI NAKATA, TAKESHI OISHI: "Synthesis of acyclic polyol derivatives via enzyme-mediated aldol reaction" TETRAHEDRON LETTERS, Bd. 34, Nr. 31, 1993, Seiten 4935-4938, XP002423415**
- **MARK D. BEDNARSKI, H. KEITH CHENAULT, ETHAN S. SIMON, GEORGE M. WHITESIDES: "Membrane-enclosed enzymatic catalysis (MEEC): A useful, practical new method for the manipulation of enzymes in organic synthesis" J. AM. CHEM. SOC., Bd. 109, 1987, Seiten 1283-1285, XP002423416**
- **DEREK HORTON, CHARNG-MING LU: "Microbial oxidation as a route for cyclization of acyclic-sugar nucleosides" CARBOHYDRATE RESEARCH, Bd. 109, 1982, Seiten 282-289, XP002423417**
- **GÜNTHER KINAST, MICHAEL SCHEDEL: "Vierstufige 1-Desoxynojirimycin-Synthese mit einer Biotransformation als zentralem Reaktionsschritt" ANGEW. CHEM., Bd. 93, Nr. 9, 1981, Seiten 799-800, XP002423418**
- **MARK D. BEDNARSKI, ETHAN S. SIMON, NORBERT BISCHOFSBERGER, WOLF-DIETER FESSNER, MAHN-JOO KIM, WATSON LEES ET AL.: "Rabbit muscle aldolase as a catalyst in organic synthesis" J. AM. CHEM. SOC., Bd. 111, 1989, Seiten 627-635, XP002423419**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 934 188 B1

- TANJA M. WRODNIGG, FREDERIK DINESS, CHRISTOPH GRUBER, HERWIG HÄUSLER, INGE LUNDT ET AL.: "Probing the aglycon binding site of a beta-glucosidase: a collection of C-1-modified 2,5-dideoxy-2,5-imino-D-mannitol derivatives and their structure-activity relationships as competitive inhibitors" BIOORGANIC & MEDICINAL CHEMISTRY, Bd. 12, 2004, Seiten 3485-3495, XP002423420
- AURELIE SEVESTRE, VIRGIL HELAINE, GHISLAIN GUYOT, CHRISTINE MARTIN, LAURENCE HECQUET: "A fluorogenic assay for transketolase from Saccharomyces cerevisiae" TETRAHEDRON LETTERS, Bd. 44, 2003, Seiten 827-830, XP002423421
- ROB SCHOEVAART, FRED VAN RANTWIJK, ROGER A. SHELDON: "Facile enzymatic aldol reactions with dihydroxyacetone in the presence of arsenate" J. ORG. CHEM., Bd. 66, 2001, Seiten 4559-4562, XP002423422
- ROBERT CHENEVERT, MOHAMMED DASSER: "Chemoenzymatic synthesis of the microbial elicitor (-)-syringolide via a fructose 1,6-diphosphate aldolase-catalyzed condensation reaction" J. ORG. CHEM., Bd. 65, 2000, Seiten 4529-4531, XP002423423
- KENTARO OKUMURA, KAZUO MATSUMOTO, MASAHARU FUKAMIZU, HARUNORI YASUO, YOSHIHIKO TAGUCHI ET AL.: "Synthesis and hypocholesterolemic activities of eritadenine derivatives" J. MED. CHEM., Bd. 17, Nr. 8, 1974, Seiten 846-855, XP002423424
- ARMANDO CORDOVA, WOLFGANG NOTZ, CARLOS F. BARBAS: "Direct organocatalytic aldol reactions in buffered aqueous media" CHEM. COMMUN., Bd. 2002, 2002, Seiten 3024-3025, XP002423425
- K. GALL MORRIS, MARK E. B. SMITH, NICHOLAS J. TURNER, MALCOLM D. LILLY, ROBIN K. MITRA, JOHN M. WOODLEY: "Transketolase from Escherichia coli: A practical procedure for using the biocatalyst for asymmetric carbon-carbon bond synthesis" TETRAHEDRON: ASYMMETRY, Bd. 7, Nr. 8, 1996, Seiten 2185-2188, XP002423426
- V. TALROSE, E.B. STERN, A.A. GONCHAROVA, N.A.MESSINEVA, N.V. TRUSOVA, M.V.EFIMKINA: ""UV/Visible Spectra" in NIST Chemistry Webbook, NIST Standard Reference Database Number 69" [Online] 2005, P.J. LINSTROM, W. G. MALLARD , NATIONAL INSTITUTE OF STANDARDS AND TECHNOLOGY, GAITHERSBURG, MD , XP002423431 Gefunden im Internet: URL:http://webbook.nist.gov/> UV/VIS spectrum of benzene

## Beschreibung

Die Erfindung betrifft UV Filter, Zubereitungen, die solche UV Filter enthalten, entsprechende Verfahren zur Herstellung der UV Filter bzw. der diese enthaltenden Zubereitungen und deren Verwendung.

Insbesondere betrifft die Erfindung die Verwendung von α,α'-Dihydroxyketon-Derivaten als Lichtschutzfilter für kosmetische oder pharmazeutische Produkte sowie neue α,α'-Dihydroxyketon-Derivate, Verfahren zu deren Herstellung und deren Verwendung in kosmetischen Zubereitungen, insbesondere zum Schutz vor Sonnenstrahlung und in pharmazeutischen Zubereitungen.

Die menschliche Haut unterliegt gewissen Alterungsprozessen, die teilweise auf intrinsische Prozesse (chronoaging) und teilweise auf exogene Faktoren (environmental, z.B. photoaging) zurückzuführen sind. Zusätzlich können vorübergehende oder auch andauernde Veränderungen des Hautbildes auftreten, wie Akne, fettige oder trockene Haut, Keratosen, Rosaceae, lichtempfindliche, entzündliche, erythematöse, allergische oder autoimmunreaktive Reaktionen wie Dermatosen und Photodermatosen.

Zu den exogenen Faktoren zählen insbesondere das Sonnenlicht oder künstliche Strahlungsquellen mit vergleichbarem Spektrum sowie Verbindungen, die durch die Strahlung entstehen können, wie undefinierte reaktive Photoprodukte, die auch radikalisch oder ionisch sein können. Zu diesen Faktoren zählen auch Zigarettenrauch und die darin enthaltenen reaktiven Verbindungen wie Ozon, freie Radikale, beispielsweise das Hydroxylradikal, Singulettsauerstoff und andere reaktive Sauerstoff- oder Stickstoffverbindungen, die die natürliche Physiologie oder Morphologie der Haut stören.

Durch den Einfluß dieser Faktoren kann es unter anderem zu direkten Schäden an der DNA der Hautzellen kommen sowie an den Kollagen-, Elastin- oder Glycosaminoglycanmolekülen der extrazellulären Matrix, die für die Festigkeit der Haut verantwortlich sind. Darüberhinaus kann es zu einer Beeinflussung der Signaltransduktionsketten kommen, an deren Ende die Aktivierung matrixabbauender Enzyme steht. Wichtige Vertreter dieser Enzyme sind die Matrixmetalloproteinasen (MMPs, z.B. Kollagenasen, Gelatinasen, Stromelysine), deren Aktivität zusätzlich durch TIMPs (tissue inhibitor of matrix metalloproteinases) reguliert wird.

Die Folgen der o.g. Alterungsprozesse sind Verdünnung der Haut, schwächere Verzahnung von Epidermis und Dermis, Reduktion der Zellzahl sowie der versorgenden Blutgefäße. Dabei kommt es zur Ausbildung von feinen Linien und Falten, die Haut wird ledrig und es können Pigmentstörungen auftreten.

Die gleichen Faktoren wirken auch auf Haare, wo es ebenfalls zu einer Schädigung kommen kann. Die Haare werden spröde, weniger elastisch und glanzlos. Die Oberflächenstruktur der Haare ist geschädigt.

Es besteht daher Bedarf nach weiteren Verbindungen, die UV-Strahlung absorbieren und so in der Lage sind, die menschliche Haut zu schützen. Durch diese Verbindungen wird die Transformation von UV-Licht in Wärme katalysiert.

Aufgrund mangelnder Hauthaftung ist aber die Schutzdauer begrenzt, insbesondere, weil konventionelle UV-Filter sehr leicht abgewaschen werden können (= begrenzte Wasserfestigkeit). Es ist eine bekannte Strategie, UV-Filter derart zu derivatisieren, dass sie über einen reaktiven Molekülpart kovalent an die Epidermis binden können. Für die effektive Anbindung an Proteine und Aminosäuren der äußeren Hautschichten ist es erforderlich, dass die entsprechenden UV-Filterderivate, bzw. Antioxidantienderivate bzw. Antioxidantienderivate mit latenter UV-Schutzwirkung über eine möglichst hohe Reaktivität ihrer bindungsfähigen Molekülteile verfügen. Idealerweise verfügen solche Stoffe oder Kombinationen solcher Stoffe zudem über die Eigenschaft, sehr breitbandig über den gesamten UV-Bereich zu schützen (UVA, B-Breitbandschutz)

Es wurde jetzt gefunden, das sich bestimmte α,α'-Dihydroxyketon-Derivate durch eine verbesserte Reaktivität ihres bindenden Molekülsteils hinsichtlich der Anbindung an Proteine und Aminosäuren auszeichnen. Diese verstärkte Anbindung führt zu verbesserten Produkteigenschaften wie Schutzdauer oder Bräunungsniveau. Zudem weisen die erfindungsgemäßen Verbindungen, insbesondere Derivate des Dihydroxyacetons und der Erythrulose, eine verbesserte Lagerstabilität auf und führen daher bei Anwendung einer kosmetischen Zubereitung zur effizienteren Anbindung an Proteine und Aminosäuren, woraus sich eine gesteigerte Schutzwirkung ableiten läßt.

Die Reaktivität solcher Verbindungen bzgl. ihrer Umsetzungen mit Proteinen und Aminosäuren der Haut im Sinne der Maillard-Reaktion wird dabei durch folgenden Molekülteil gesteuert:

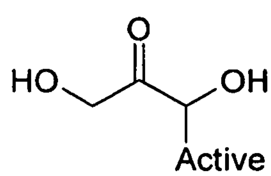

Gegenstand der Erfindung sind Verbindungen nach Anspruch 1 der Formel II

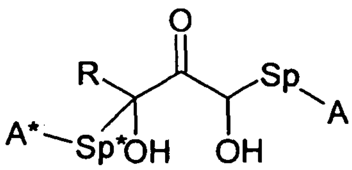

II

worin

- R steht für H oder $(CHOH)_nCH_2OH$ (n= 0, 1 oder 2),
- Sp, Sp* stehen für -CH-, -CHOH-, $-(CH_2)_n-$, $-(CH=CH)_n-$, $-(CH=CH-CH_2)_n-$, - $(CH_2)_n-X-(CH_2)_o-$, $-(CH_2-X)_n-(CH_2)_o-$, $-(CH_2)_n-C(=X)-(CH_2)_o-X-(CH_2)_p-$,$-(CH_2)_n-X-(CH_2)_o-C(=X)-(CH_2)_p-$, $-(CH_2)_n-C(=X)-(CH_2)_o-$, und ist über eine Einfach- oder Doppelbindung an das in Richtung der Carbonylgruppe benachbarte C-Atom gebunden,
- n steht für eine ganze Zahl unabhängig voneinander ausgewählt aus dem Bereich beginnend mit 0 und endend mit 20,
- X steht für O, N oder S,
- A, A* stehen unabhängig voneinander für einen Substituenten der UV-Strahlung absorbiert und ein konjugiertes $\pi$-Elektronensystem von mindestens $4\pi$-Elektronen aufweisen,
- wobei A und A* für gleiche oder verschiedene Reste stehen können, und Verbindungen nach Anspruch 4 der Formeln Ia-h.

Bevorzugt sind Verbindungen der Formel II, in denen R für ein Wasserstoffatom steht.

Zubereitungen, die mindestens eine Verbindung gemäß den Formeln Ia-h oder II enthalten sind ein weiterer Erfindungsgegenstand.

Die Verwendung mindestens einer Verbindung gemäß den Formeln Ia-h oder II oder einer Zubereitung, die mindestens eine Verbindung gemäß Formel Ia-h oder II enthält zur Pflege, Konservierung oder Verbesserung des allgemeinen Zustands der Haut oder Haare sowie vorzugsweise zur Prophylaxe gegen zeit- und/oder lichtinduzierte Alterungsprozesse der menschlichen Haut oder menschlicher Haare, insbesondere zur Prophylaxe gegen trockene Haut, Faltenbildung und/oder Pigmentstörungen, und/oder zur Reduktion oder Verhinderung schädigender Effekte von UV-Strahlen auf die Haut und zur Prophylaxe gegen oder Reduktion von Hautunebenheiten, wie Falten, feinen Linien, rauher Haut oder großporiger Haut sind entsprechend den vorteilhaften Eigenschaften der erfindungsgemäßen Verbindungen sind weitere Gegenstände der vorliegenden Erfindung.

Die herkömmlichen Lichtschutzfilter weisen in der Regel eine geringe oder eine ungenügende Hauthaftung auf, was zu einer kürzeren Dauer der Schutzwirkung des Filters und insbesondere zur nahezu vollständigen Entfernung des Filters beim Baden führt.

Es erscheint somit weiter wünschenswert, Verbindungen zur Verfügung zu stellen, welche die menschliche Haut gegen UV-Strahlen lange schützen können.

Die vorliegende Erfindung löst dieses Problem mit den erfindungsgemäß bevorzugten Verbindungen nach Anspruch 3 und 4. Diese erfindungsmäßigen Verbindungen sind in der Lage mit der Haut chemisch zu verknüpfen.

Es ist bekannt, dass die $\alpha,\alpha'$-Dihydroxyketon-Derivate über eine vorzügliche Hauthaftung und in einigen Fällen über einen Selbstbräunungseffekt verfügen, wie z.B. andere Dihydroxyketoverbindungen (Dihydroxyaceton).

Aus der EP 0 758 314 B1 sind Maleimide und Maleinsäure Derivate bekannt, die mit in der Haut vorhandenen SH-Gruppierungen reagieren können.

Aus der US 6 613 341 B2 sind $\alpha,\beta$-ungesättigte Ketone bekannt, die mit UV-Filtern bzw. Antioxidationsmitteln chemisch verknüpft sind.

Aus der EP 0 581 954 B1 sind $\alpha$-Hydroxyketoalkyl-Derivate bekannt.

Aus der WO 2001085124 sind verschiedene silylierte Verbindungen (unter anderem das silylierte DHA) bekannt, die als "precursor" für Selbstbräunungsmittel in kosmetischen Formulierungen eingesetzt werden können.

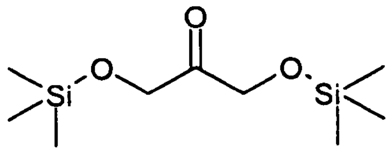

Aus der EP 796838 A1 sind DHA-Carbonate bekannt, die als Selbstbräunungsmittel in kosmetischen Formulierungen verwendet werden können.

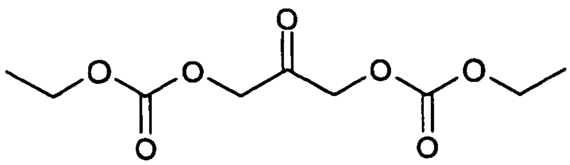

Aus der EP 710478 A1 und EP 709081 A1 sind DHA-Fettsäure-Ester bekannt, die zusammen mit Lipase als Selbstbräunungsmittel in Cremen verwendet werden können.

Aus DE 19720831 sind einige DHA-Ester bekannt, die als Selbstbräunungsmittel und Sunscreens verwendet werden können.

In EP 1481663 A1 werden DHA-Derivate und deren Verwendung als Selbstbräuner und Hautpflegemittel offenbart.

In einer Gruppe von bevorzugt als UV-Filter einzusetzenden Verbindungen nach den Formeln Ia-h nach Anspruch 4 oder II nach Anspruch 1 handelt es sich insbesondere bevorzugt um eine Verbindung aus der folgenden Gruppe:

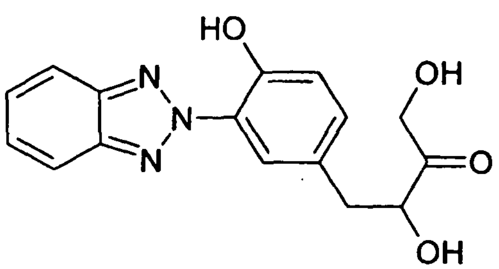

Ia

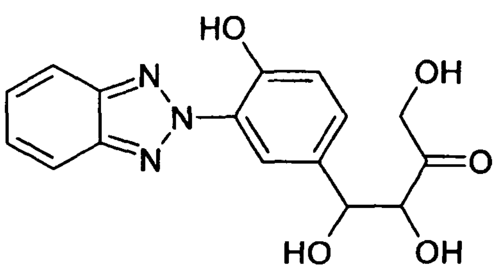

Ib

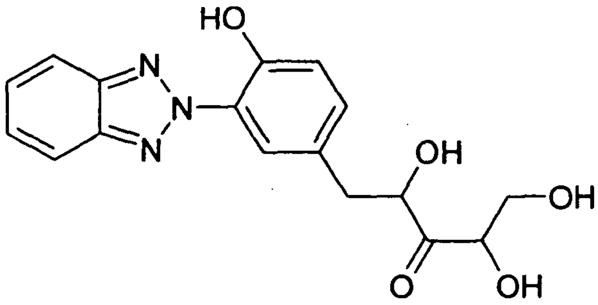

Ic

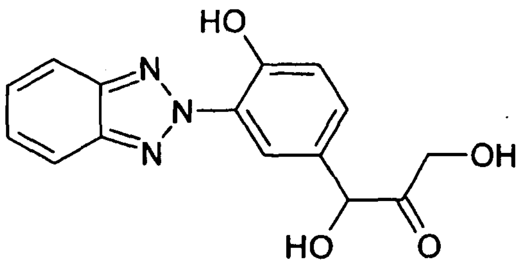

Id

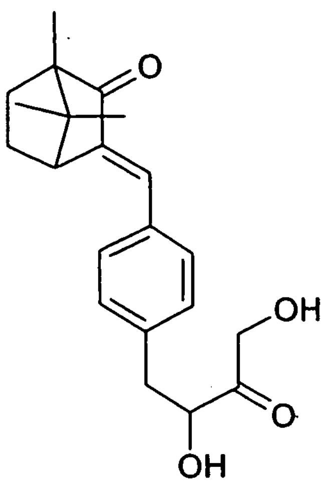

Ie

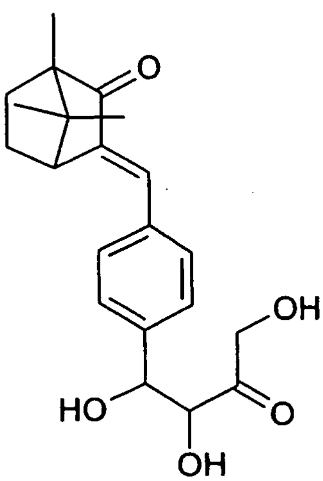

If

Ig

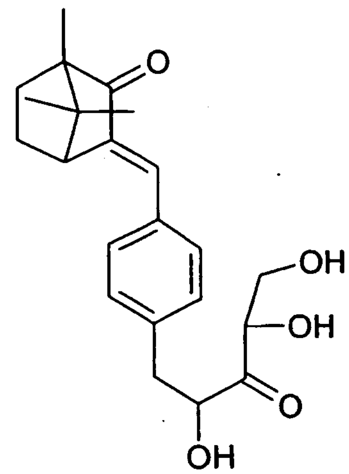

Ih

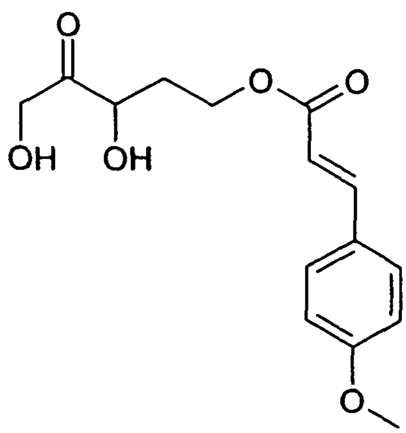

IIa

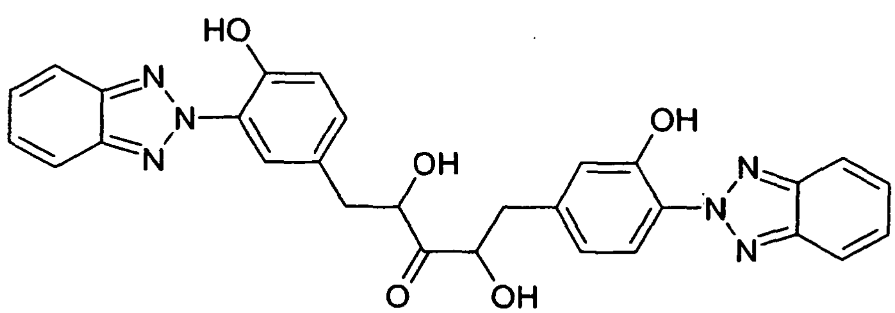

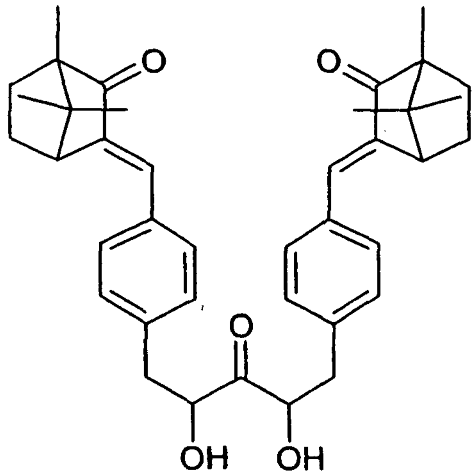

IIb

Besonders bevorzugt ist dabei die Verbindung If.

Enthaltene nicht-aromatische Doppelbindungen liegen gegebenenfalls in hydrierter Form vor. Sind mehrere nicht-aromatische Doppelbindungen im Molekül enthalten, dann liegen gegebenenfalls einzelne oder alle nicht-aromatische Doppelbindungen in hydrierter Form vor.

Die erfindungsgemäßen Verbindungen können auch in dimerer Form vorliegen.

Die erfindungsmäßigen Verbindungen können, je nach Chromophor "A" bzw. "A*", nach verschiedenen Syntheseprinzipien dargestellt werden.

So ist ein Verfahren zur Herstellung von Verbindungen gemäß den Formeln Ia-h oder II, dadurch gekennzeichnet, dass ein Dihydroxyaceton-Derivat (DHA-Derivat), welches mit einer Base wie Lithiumdiisopropylamid (LDA) oder Lithiumhexamethyldisilazan (Li-HMDS) deprotoniert wird, mit einem UV-Filterhalogenid oder -aldehyd umgesetzt und anschließend mit Salzsäure hydrolysiert und/oder gegebenenfalls mit einer Base wie Ammoniak gespalten wird.

Bevorzugt wird als DHA-Derivat ein DHA-Dibenzoylester, -Dibenzoylesterimin, -Acetal oder -Orthoester eingesetzt. Noch bevorzugter wird als DHA-Derivat ein DHA-Acetal (z.B. DHA-Acetat) oder ein DHA-Orthoester (z.B. DHA-Orthoacetat) eingesetzt.

Bevorzugt wird als UV-Filterahalogenid oder -aldehyd ein ortho-tert.-Butyldimethylsilylbenzotriazolbromid, ortho-tert.-Butyldimethylsilylbenzotriazolaldehyd, Zimtsäurechlorethylester, Methylbenzylidencampherbromid oder Methylbenzylidencampheraldehyd eingesetzt.

Die Synthese der UV-Filter-Edukte (entspricht in den Formeln I und II den Resten Sp-A bzw. Sp-A*) erfolgt bevorzugt nach vier unterschiedlichen, in der Literatur bekannten, Methoden:

- Ein Zimtsäurederivat (z.B. p-Methoxyzimtsäure) reagiert mit Chlorethanol als Lösungsmittel und einer Säure (z.B. Schwefelsäure) als Katalysator unter Bildung eines entsprechenden Esters (z.B. zum Zimtsäurechlorethylester), [Chem. Berichte 1951, 84, 734-744]

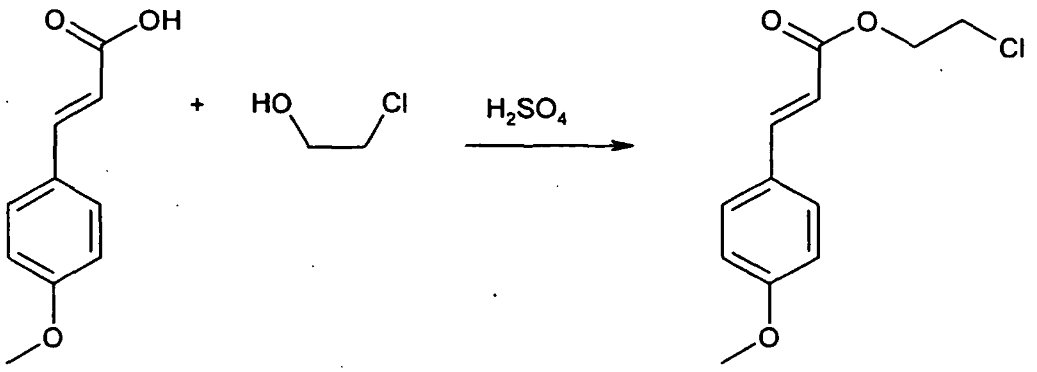


- In einem weiteren bekannten Verfahren (siehe US 6,284,895) wird ein Benzotriazolderivat bromiert und anschließend silyliert.

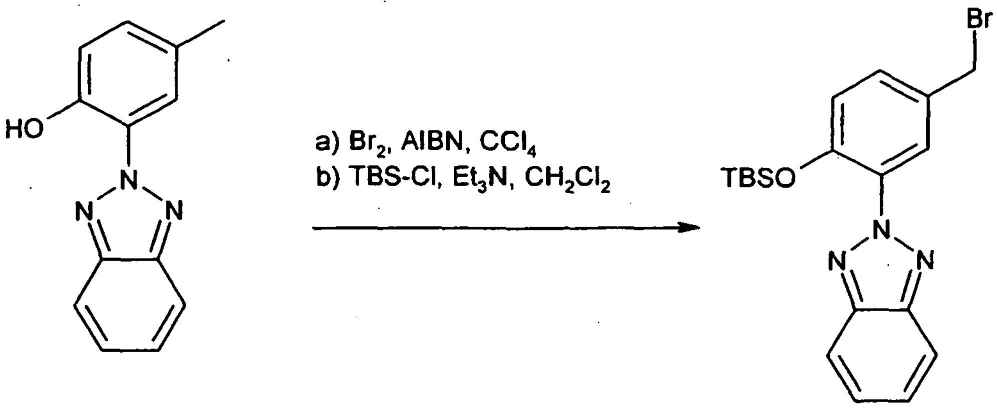


- Bei einer dritten bekannten Verfahrensvariante (siehe DE 3445365) wird ein Campherderivat (z.B. Methylbenzylidencampher) mittels N-Bromsuccinimid bromiert.

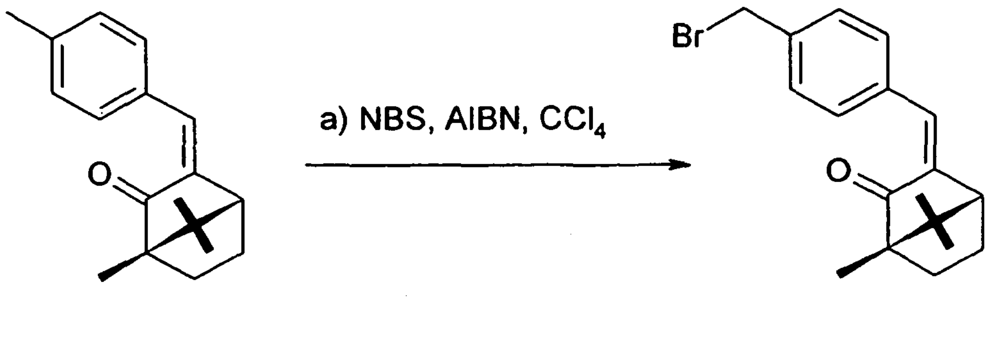


- Besonders bevorzugt ist eine vierte Verfahrensvariante zur Darstellung eines UV-Filter-Eduktes bei der Campher mit einem Acetal (z.B. Terephthalidenaldehyd-Monoethylenacetal) zum entsprechenden Campheraldehyd umgesetzt wird.

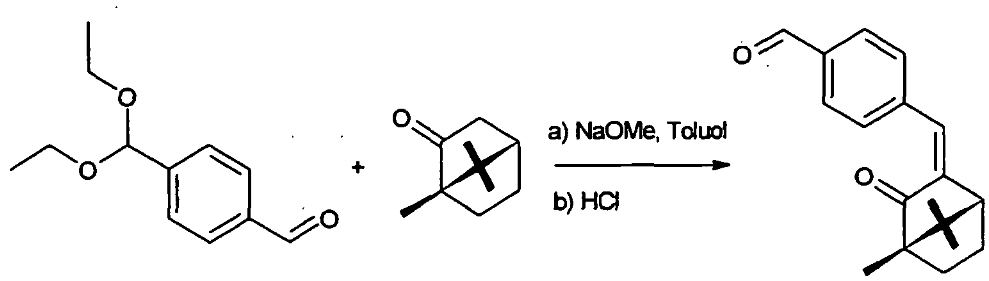


Das DHA-Derivat bzw. DHA-Zucker-Edukt kann ebenfalls nach vier verschiedenen (teils bekannten) Methoden hergestellt werden:

- DHA (dimer) wird mittels Pyridin monomerisiert und mit einem Säurechlorid (z. B. Benzoylchlorid) zu einem DHA-Dibenzoylester umgesetzt.

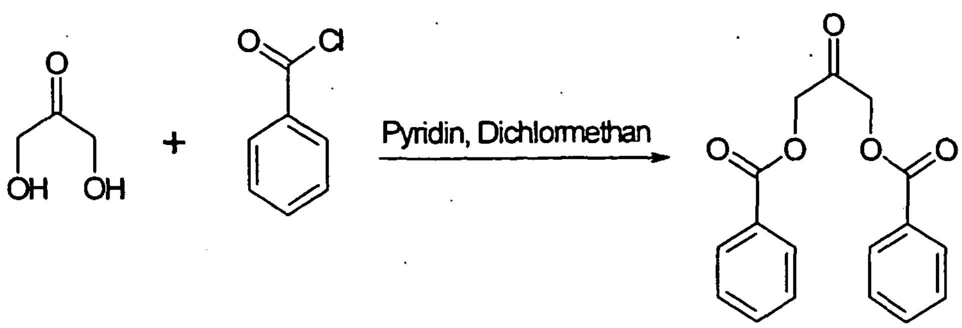


- Alternativ kann der vorher synthetisierte DHA-Dibenzoylester auch mit einem Amin (z.B. Cyclohexylamin) zum ensprechenden Imin-Derivat umgesetzt werden.

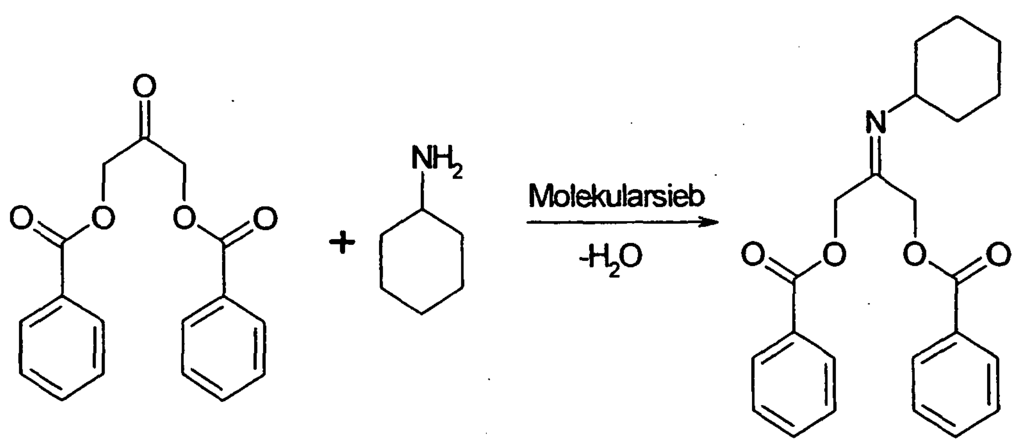


- Eine dritte bekannte Alternative (Tetrahedron, 1989, 45,3, 687-694) geht von einem Triol aus, welches mit einem Katalysator (z.B. PTSA=p-Toluolsulfonsäure) und 2,2 Dimethoxypropan zum Acetonid umgesetzt wird. Dieses wird dann mit einem Phosphat (z.B. Kaliumhydrogenphosphat) und Natriumperiodat oxidativ zum DHA-Acetonid (z.B. 2,2-Dimethyl-1,3-dioxon-5-on) gespalten.

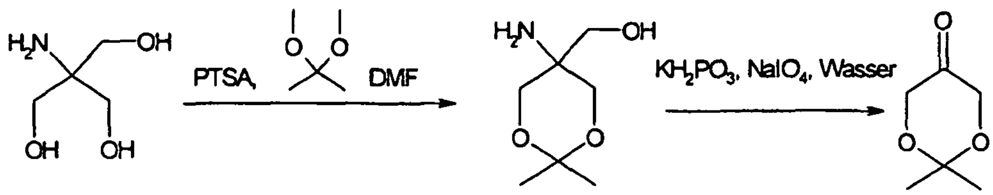


- Bei einer weiteren besonders bevorzugten Verfahrensvariante (JACS, 1997,119, 2795-2803) wird DHA mit Camphersulfonsäure (CSA) als Katalysator und einem Acetat (z.B. Trimethoxyorthoacetat) umgesetzt, so dass ein DHA-Orthoester (z.B. 2-Methyl-2-methoxy-1,3-dioxan-5-on) entsteht.

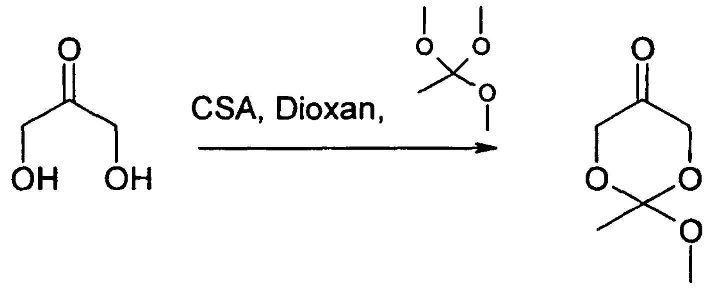


Für die Hauptstufen-Synthesen, in denen ein UV-Filter-Edukt (-halogenid oder -aldehyd) mit einem DHA-Zucker-Edukt (vorzugsweise DHA-Ester - Imin, -Acetonid oder -orthoester) zu den Verbindungen gemäß den Formeln Ia-h und II reagiert, sind folgende vier Varianten bevorzugt, wobei die vierte Synthesevariante besonders bevorzugt ist:

- Bei ersten Hauptstufensynthese-Variante wird ein DHA-Derivat (z.B. ein DHA-Dibenzoylester) mit einer Base (z.B. LDA oder Li-HDMS) deprotoniert und mit einem UV-Filterhalogenid oder -aldehyd (z.B. o-tert.Butyldimethylsilylbenzotriazolbromid oder -aldehyd, Zimtsäurechlorethylester, Methylbenzylidencampherbromid oder - aldehyd) umgesetzt. Anschließend erfolgt die Spaltung des Dibenzoylesters mit Ammoniak.

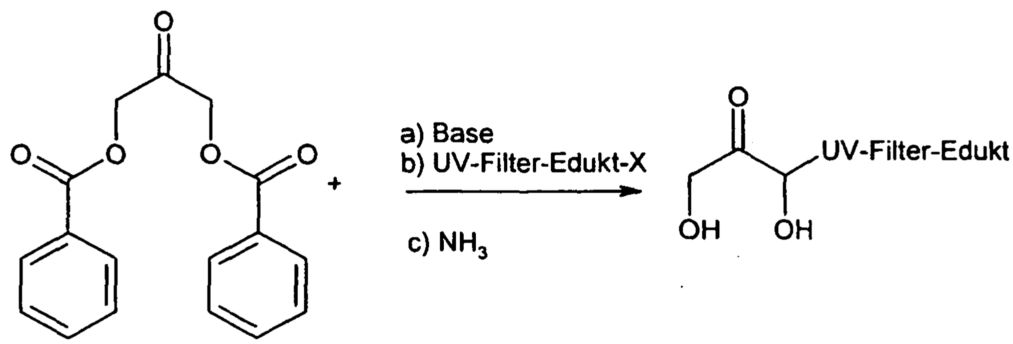


- Die zweite Hauptstufensynthese-Variante erfolgt analog der ersten Variante, mit dem Unterschied, dass vor dem letzten Schritt nämlich der Spaltung des Dibenzoylesters mit $NH_3$, noch eine saure Hydrolyse des Imins erfolgt.

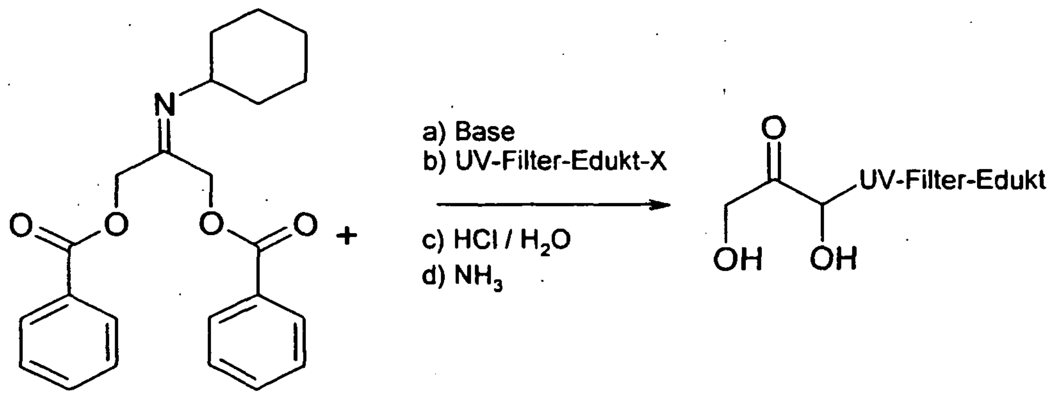


- Bei einer weiteren Hauptstufensynthese-Variante wird ein DHA-Acetal (z.B. DHA-Acetonid) mit einer Base (z.B. LDA oder Li-HDMS) deprotoniert und mit einem UV-Filterhalogenid oder -aldehyd (z.B. o-tert.Butyldimethylsilylbenzotriazolbromid oder -aldehyd, Zimtsäurechlorethylester, Methylbenzylidencampherbromid oder - aldehyd) umgesetzt. Anschließend erfolgt die saure Spaltung des Acetals mit Salzsäure.

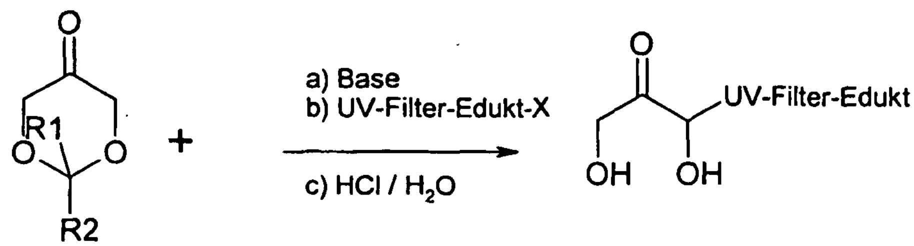


R1, R2 = gleich oder verschieden, bestehend z.B. aus H, Methyl, Ethyl, Alkyl, Phenyl, Aryl

- Die vierte, besonders bevorzugte Hauptstufensynthese-Variante, ausgehend von einem DHA-Orthoester (z.B. DHA-orthoacetat), erfolgt analog zur dritten Synthesevariante.

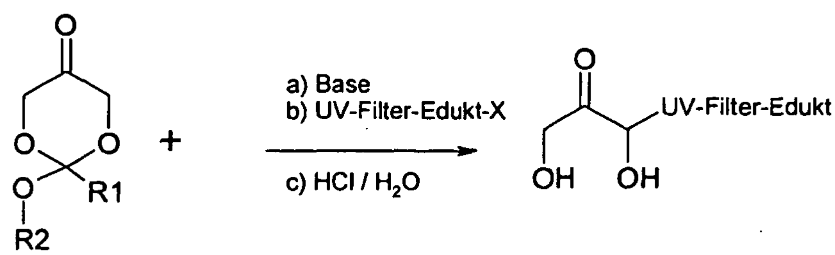


R1, R2 = gleich oder verschieden, bestehend z.B. aus H, Methyl, Ethyl, Alkyl, Phenyl, Aryl

Um die Verbindungen nach ForMel II herzustellen muss im Gegensatz zu den Verbindungen nach Formel Ia-h in Bezug auf das DHA-Edukt-Derivat eine doppelte Menge Base und UV-Filter-Derivat eingesetzt werden.

Ein weiterer Gegenstand der vorliegenden Erfindung sind Verbindungen nach Formel III

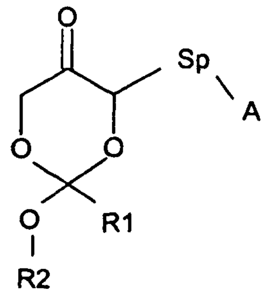

III

worin

- R1, R2 gleich oder verschieden sind und H, Alkyl, Phenyl oder Aryl bedeuten,
- Sp steht für -CH-, -CHOH-, $-(CH_2)_n-$, $-(CH=CH)_n-$, $-(CH=CH-CH_2)_n-$, $-(CH_2)_n-X-(CH_2)_o-$, $-(CH_2-X)_n-(CH_2)_o-$, $-(CH_2)_n-C(=X)-(CH_2)_o-X-(CH_2)_p-$, $-(CH_2)_n-X-(CH_2)_o-C(=X)-(CH_2)_p-$, $-(CH_2)_n-C(=X)-(CH_2)_o-$, und ist über eine Einfach- oder Doppelbindung an das in Richtung der Carbonylgruppe benachbarte C-Atom gebunden,
- n, p, o stehen für eine ganze Zahl, unabhängig voneinander ausgewählt aus dem Bereich beginnend mit 0 und endend mit 20,
- X steht für O, N oder S,
- A steht unabhängig voneinander für einen Substituenten der UV-Strahlung absorbiert und ein konjugiertes $\pi$-Elektronensystem von mindestens $4\pi$-Elektronen aufweist.

wobei A für gleiche oder verschiedene Reste stehen kann.

Die erfindungsgemäßen Verbindungen gemäß Formel III eignen sich zum einen selbst als UV-Filter und sind zum anderen wertvolle Zwischenstufen bei der Synthese von Verbindungen gemäß Formel Ia-h oder II.

Gemeint sind dabei die UV-Filteraddukte an DHA-Dibenzoylester, DHA-Dibenzoylesterimin, DHA-Acetonid, DHA-Methylorthoester oder DHA-Ethylorthoester vor der abschließenden Hydrolyse. Diese Zwischenstufen könnten erst in-situ auf der Haut hydrolytisch (Haut-pH ca. 5) in die eigentliche hautbindende Komponente überführt werden. Diese Zwischenstufen sind u.U. chemisch stabiler als die eigentliche bindende und chemisch sehr reaktive Komponente gemäß den Formeln Ia-h und II.

In einer Gruppe von bevorzugt als Synthesezwischenstufe einzusetzenden Verbindungen nach Formel III handelt es sich insbesondere bevorzugt um einen DHA-Orthoester aus der folgenden Gruppe:

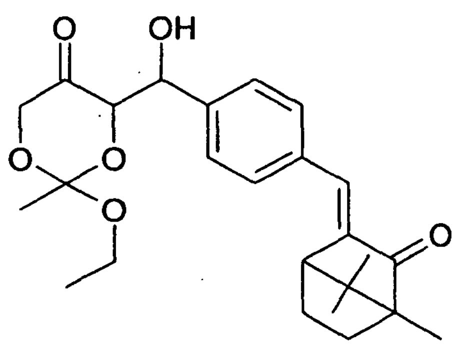

IIIa

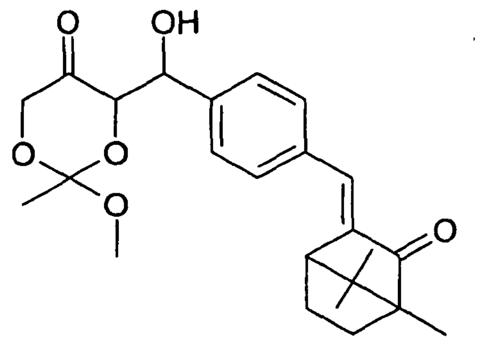

IIIb

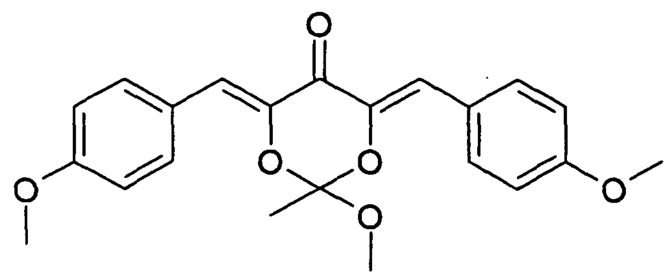

IIIc

Entsprechend können auch Verbindungen gemäß Formel III, hergestellt werden, wobei das Verfahren analog zu den vorher beschriebenen Verfahren verläuft, nur mit dem Unterschied, dass jeweils der letzte Schritt der Hydrolyse bei der Hauptstufensynthese fehlt.

Ein weiterer Gegenstand der vorliegenden Erfindungen sind Zubereitungen enthaltend einen geeigneten Träger, dadurch gekennzeichnet, dass die Zubereitung 0,001 bis 99 Gew.-% mindestens einer Verbindung nach den Formeln Ia-h, II oder III oder deren topisch verträglicher Salze und/oder Derivate enthält. Dabei sind eine oder mehrere Verbindungen der Formel Ia-h, II oder III vorzugsweise in Mengen von 0,01 bis 20 Gew.-%, vorzugsweise 0,05 bis 10 Gew.-% und insbesondere bevorzugt 0,1 bis 5 Gew.-% in der Zubereitung enthalten.

In einer bevorzugten Ausführungsform der vorliegenden Erfindung handelt es sich bei der Zubereitung um eine Zubereitung zum Schutz von Körperzellen gegen oxidativen Stress, insbesondere zur Verringerung der Hautalterung, dadurch gekennzeichnet, dass sie neben einer oder mehrere Verbindungen nach Formel Ia-h, II oder III ein oder mehrere weitere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B.

Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäure$_n$, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der Formel I oder Formel II in solchen Zusammensetzungen überlicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-acetat, Nicotinsäure, Pantothensäure und Biotin. Vitamine werden dabei mit Verbindungen der Formel I, II oder Formel III überlicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind weiter Verbindungen der Formel IV

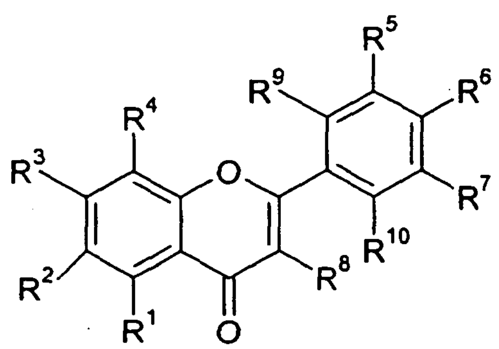

IV

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,

- geradkettigen oder verzweigten $C_3$- bis $C_{20}$-Alkenylgruppen,

- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder

- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,

- wobei alle $OR^{11}$ unabhängig voneinander stehen für

  - OH

  - geradkettige oder verzweigte $C_1$- bis $C_{20}$-Alkyloxygruppen,

  - geradkettigen oder verzweigten $C_3$- bis $C_{20}$-Alkenyloxygruppen,

  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder

  - $C_3$- bis $C_{10}$-Cycloalkyloxygruppen und/oder $C_3$- bis $C_{12}$-Cyclolkenyloxygruppen, wobei die Ringe jeweils auch durch $-(CH_2)_n$- Gruppen mit n = 1 bis 3 überbrückt sein können und/oder,

  - Mono- und/oder Oligoglycosylreste,

  mit der Maßgabe, dass mindestens 4 Reste aus $R^1$ bis $R^7$ stehen für OH und dass im Molekül mindestens 2 Paare benachbarter Gruppen -OH vorliegen,

- oder $R^2$, $R^5$ und $R^6$ für OH und die Reste $R^1$, $R^3$, $R^4$ und $R^{7-10}$ für H stehen,

wie sie in der Deutschen Patentanmeldung DE-A-10244282 beschrieben sind.

Erfindungsgemäß bevorzugte Zubereitungen können neben den Verbindungen der Formel Ia-h, II bzw. Formel III auch weitere UV-Filter enthalten.

Prinzipiell kommen alle UV-Filter für eine Kombination mit den erfindungsgemäßen Verbindungen der Formel Ia-h, II bzw. Formel III in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z. B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),

- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und

- 2,4,6-Trianilino-(p-carbo-2'-ethylhexyl-1'-oxi)-1,3,5-triazin (z.B. Uvinul® T 150)

- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.
Diese organischen UV-Filter werden in der Regel in einer Menge von 0,5 bis 10 Gewichtsprozent, vorzugsweise 1 - 8 %, in kosmetische Formulierungen eingearbeitet.
Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),

- 4,4'-[(6-[4-((1,1-Dimethylethyl)aminocarbonyl)phenylaminoJ-1,3,5-triazin-2,4-diyl)diimino]bis(benzoesäure-2-ethylhexylester) (z.B. Uvasorb® HEB),

- α-(Trimethylsilyl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)

- 2,2'-Methylen-bis-(6-(2H-benzotriazol-2-yl)-4-(1,1,3,3-tetramethylbutyl)phenol) (CAS-Nr. 103 597-45-1)

- 2,2'-(1,4-Phenylen)bis-(1H-benzimidazol-4,6-disulfonsäure, Mononatriumsalz) (CAS-Nr. 180 898-37-7) und

- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Weitere geeignete UV-Filter sind auch Methoxyflavone ensprechend der älteren Deutschen Patentanmeldung DE-A-10232595.
Organische UV-Filter werden in der Regel in einer Menge von 0,5 bis 20 Gewichtsprozent, vorzugsweise 1 - 15 %, in kosmetische Formulierungen eingearbeitet.
Als anorganische UV-Filter sind solche aus der Gruppe der Titandioxide wie z.B. gecoatetes Titandioxid (z.B. Eusolex® T-2000, Eusolex®T-AQUA, Eusolex® T-AVO), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.
Bevorzugte Verbindungen mit UV-filternden Eigenschaften sind 3-(4'-Methylbenzyliden)-dl-campfer, 1-(4-tert-Butylphenyl)-3-(4-methoxy-phenyl)-pro-pan-1,3-dion, 4-Isopropyldibenzoylmethan, 2-Hydroxy-4-methoxybenzophenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.
Durch Kombination von einer oder mehrerer Verbindungen der Formel Ia-h, II bzw. Formel III mit weiteren UV-Filtern kann die Schutzwirkung gegen schädliche Einwirkungen der UV-Strahlung optimiert werden.
Optimierte Zusammensetzungen können beispielsweise die Kombination von UVA mit UV-Filtern gemäß Formel Ia-h, II bzw. III enthalten. Mit dieser Kombination ergibt sich ein Breitbandschutz, der durch Zusatz von anorganischen UV-Filtern, wie Titandioxid-Mikropartikeln noch ergänzt werden kann.
Alle genannten UV-Filter können auch in verkapselter Form eingesetzt werden. Dabei können auch die Verbindungen der Formeln Ia-h, II bzw. III in verkapselter Form eingesetzt werden. Dabei ist es allerdings bevorzugt, die hauthaftenden Verbindungen der Formel Ia-h, II bzw. III in unverkapselter Form einzusetzen. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der

häufig als unangenehm empfundene ölige Eindruck beim Auftragen der hydrophobe UV-Filter enthaltenden Zubereitung unterbunden.

- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.

- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisations-vorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter und / oder der Verbindungen nach Formel Ia-h, II oder III in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Siliciumoxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Dabei kann es erfindungsgemäß bevorzugt sein, wenn die Zubereitungen die Hautpenetration erleichternde Wirkstoffe, sogenannte "Penetrationsenhancer" enthalten. Diese die Hautpenetration erleichternden Wirkstoffe können bewirken, dass die erfindungsgemäßen hauthaftenden UV Filter in tiefere Hautschichten vordringen, die erst nach beträchtlicher Zeit abgestoßen werden und dadurch einen besonders langandauernden UV-Schutz zu Verfügung stellen. Als geeignete Enhancer werden in der Literatur unterschiedliche Substanzen beschrieben, die in drei Klassen eingeteilt werden (Lambert WJ, Kudlar RJ, Hollard JM, Curry JT (1993) Int J Pharm, 45:181): Lösungsmittel mit H-Bindungsakzeptoren, einfache Fettsäuren und Alkohole und schwach oberflächenaktive Stoffe. Eine chemische Einteilung unterscheidet zwischen Alkoholen, Sulfoxiden, Fettsäuren, Fettsäure-estern, Polyolen, Tensiden, Terpenen und organischen Säuren (Kalbitz J, Neubert R, Wohlrab W (1996) Modulation der Wirkstoffpenetration in die Haut. Pharmazie, 51:619-637). Ethanol und 1,2-propandiol gehören dabei zu den erfindungsgemäß besonders bevorzugten die Hautpenetration erleichternden Wirkstoffen.

Die erfindungsgemäßen Zubereitungen können darüber hinaus weitere übliche hautschonende oder hautpflegende Wirkstoffe enthalten. Dies können prinzipiell alle dem Fachmann bekannten Wirkstoffe sein.

Weiter kann es bevorzugt sein, wenn die erfindungsgemäße Zubereitung mindestens ein Repellent enthält, wobei das Repellent vorzugsweise ausgewählt ist aus N,N-Diethyl-3-methylbenzamid, 3-(Acetyl-butyl-amino)-propionsäure Ethylester, Dimethylphthalat, Butopyronoxyl, 2,3,4,5-bis-(2-Butylen)-tetrahydro-2-furaldehyd, N,N-Caprylsäurediethylamid, N,N-Diethylbenzamid, o-Chlor-N,N-diethylbenzamid, Dimethylcarbat, Di-n-propylisocinchomeronat, 2-Ethylhexan-1,3-diol, N-Octyl-bi-cyclohepetendiecarboximid, Piperonyl-butoxid, 1-(2-Methylpropyloxycarbonyl)-2-(hydroxyethyl)-piperidin oder Mischungen davon, wobei es insbesondere bevorzugt ausgewählt ist aus N,N-Diethyl-3-methylbenzamid, 3-(Acetylbutyl-amino)-propionsäure-ethylester 1-(2-Methytpropyloxycarbonyl) 2-(hydroxyethyl)-piperidin oder Mischungen davon.

Bei den erfindungsgemäßen Zubereitungen, die Repellentien enthalten, handelt es sich dabei vorzugsweise um Insektenabwehrmittel. Insektenabwehrmittel werden in Form von Lösungen, Gelen, Stiften, Rollern, Pump-Sprays und Aerosol-Sprays angeboten, wobei Lösungen und Sprays den Hauptteil der im Handel erhältlichen Produkte bilden. Basis für diese beiden Produktformen sind meist alkoholische bzw. wässrigalkoholische Lösungen unter Zusatz fettender Substanzen und leichter Parfümierung.

Besonders bevorzugte Wirkstoffe sind beispielsweise auch sogenannte kompatible Solute. Es handelt sich

dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden:

Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1- Diglycerin-Phosphat (DGP), β-Mannosylglycerat (Firoin), β- Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Tetrahydropyrimidincarbonsäure gemäß der unten stehenden Formel V eingesetzt,

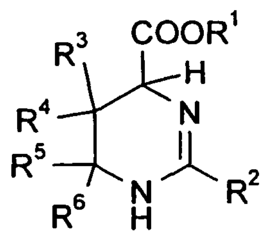

V

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den Verbindungen der Formel Ia-h, II oder III eingesetzt, wobei Verhältnisse im Bereich 1:10 bis 10:1 besonders bevorzugt sind.

Erfindungsgemäß insbesondere bevorzugt ist es dabei, wenn die kompatiblen Solute ausgewählt sind aus Di-myo-inositol-phosphat (DIP), cyclisches 2,3-Diphosphoglycerat (cDPG), 1,1- Diglycerin-Phosphat (DGP), β-Mannosylglycerat (Firoin), β- Mannosylglyceramid (Firoin-A) oder/und Di-mannosyl-di-inositolphosphat (DMIP), Ectoin, Hydroxyectoin oder Mischungen davon.

Unter den ebenfalls bevorzugt eingesetzten Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel Ia-h, II oder III zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

In einer weiteren ebenfalls bevorzugten Ausführungsform der vorliegenden Erfindung enthält die erfindungsgemäße Zubereitung mindestens einen Selbstbräuner.

Als vorteilhafte Selbstbräuner können unter anderem eingesetzt werden:

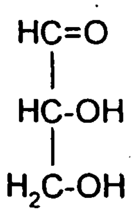

Glycerolaldehyd

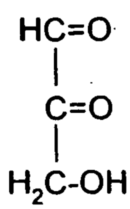

Hydroxymethylglyoxal

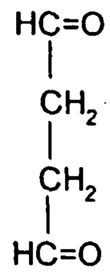

Succindialdehyd

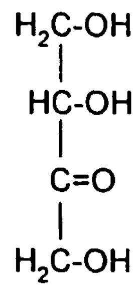

Erythrulose

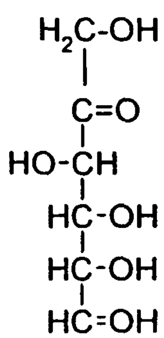

6-Aldo-D-Fructose

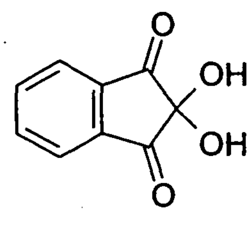

Ninhydrin

Ferner ist das 5-Hydroxy-1,4-naphtochinon (Juglon) zu nennen, das aus den Schalen frischer Walnüsse extrahiert wird

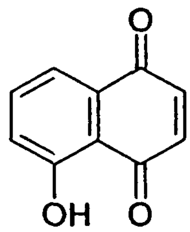

5-Hydroxy-1,4-naphtochinon (Juglon)

sowie das in den Henna-Blättern vorkommende 2-Hydroxy-1,4-naphtochinon (Lawson).

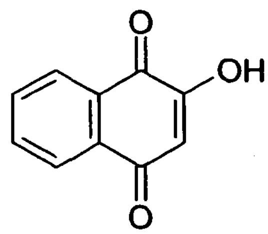

2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

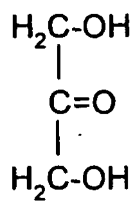

1,3-Dihydroxyaceton (DHA)

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-sulfonsäure | 10316 | gelb |
| Pigment Yellow 1 | 11680 | gelb |

(fortgesetzt)

| Chemische oder sonstige Bezeichnung | CIN | Farbe |
|---|---|---|
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'sulfonsäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfonsäure | 13015 | gelb |
| 2,4-Dihydroxy-azobenzol-4'-sulfonsäure | 14270 | orange |
| 2-(2,4-Dimethylphenylazo-5-sulfonsäure)-1-hydroxynaphthalin-4-sulfonsäure | 14700 | rot |
| 2-(4-Sulfo-1-naphthylazo)-1-naphthol-4-sulfonsäure | 14720 | rot |
| 2-(6-Sulfo-2,4-xylylazo)-1-naphthol-5-sulfonsäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfonsäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfonsäure | 15630 | rot |
| 3-Hydroxy-4-phenylazo-2-naphtalincarbonsäure | 15800 | rot |
| 1-(2-Sulfo-4-methyl-1-phenylazo)-2-naphthalincarbonsäure | 15850 | rot |
| 1-(2-Sulfo-4-methyl-5-chlor-1-phenylazo)-2-hydroxynaphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfonsäure | 15980 | orange |
| 1-(4-Sulfo-1-phenylazo)-2-naphthol-6-sulfonsäure | 15985 | gelb |
| Allura Red | 16035 | rot |
| 1-(4-Sulfo-1-naphthylazo)-2-naphthol-3,6-disulfonsäure | 16185 | rot |
| Acid Orange 10 | 16230 | orange |
| 1-(4-Sulfo-1-naphthylazo)-2-naphthol-6,8-disulfonsäure | 16255 | rot |
| 1-(4-Sulfo-1-naphthylazo)-2-naphthol-3,6,8-trisulfonsäure | 16290 | rot |
| 8-Amino-2-phenylazo-1-naphthol-3,6-dinsulfonsäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxypyrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |

(fortgesetzt)

| Chemische oder sonstige Bezeichnung | CIN | Farbe |
|---|---|---|
| Solvent Yellow | 21230 | gelb |
| Acid Red 163 | 24790 | rot |
| Acid Red 73 | 27290 | rot |
| 2-[4'-(4''-Sulfo-1''-phenylazo)-7'-sulfo-1'-naphthylazo]-1-hydroxy-7-aminonaphthalin-3,6-disulfonsäure | 27755 | schwarz |
| 4-[4''-Sulfo-1''-phenylazo)-7'-sulfo-1'-naphthylazo]-1-hydroxy-8-acetyl-aminonaphthalin-3,5-disulfonsäure | 28440 | schwarz |
| Direct Orange 34, 39, 44, 46, 60 | 40215 | orange |
| Food Yellow | 40800 | orange |
| trans-β-Apo-8'-Carotinaldehyd ($C_{30}$) | 40820 | orange |
| trans-Apo-8'-Carotinsäure ($C_{30}$)-ethylester | 40850 | orange |
| Canthaxanthin | 40850 | orange |
| Acid Blue 1 | 42045 | blau |
| 2,4-Disulfo-5-hydroxy-4'-4''-bis-(diethylamino)triphenylcarbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | blau |
| Acid Green 9 | 42100 | grün |
| Diethyl-di-sulfobenzyl-di-4-amino-2-chlor-di-2-methylfuchsonimmonium salz | 42170 | grün |
| Basic Violet 14 | 42510 | violet |
| Basic Violet 2 | 42520 | violet |
| 2'-Methyl-4'-(N-ethyl-N-m-sulfobenzyl)-amino-4''-(N-diethyl)-amino-2-methyl-N-ethyl-N-m-sulfobenzylfuchsonimmonium salz | 42735 | blau |
| 4'-(N-Dimethyl)-amino-4''-(N-phenyl)-aminonaphtho-N-dimethylfuchsonimmonium salz | 44045 | blau |
| 2-Hydroxy-3,6-disulfo-4,4'-bis-dimethylaminonaphthofuchsonimmonium salz | 44090 | grün |
| Acid Red 52 | 45100 | rot |
| 3-(2'-Methylphenylamino)-6-(2'-methyl-4'-sulfophenylamino)-9-(2''-carboxyphenyl)-xanthenium salz | 45190 | violet |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfonsäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-trisulfonsäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |

(fortgesetzt)

| Chemische oder sonstige Bezeichnung | CIN | Farbe |
|---|---|---|
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfonsäure | 73015 | blau |
| 4,4'-Dimethyl-6,6'-dichlorthioindigo | 73360 | rot |
| 5,5'Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin . | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |

(fortgesetzt)

| Chemische oder sonstige Bezeichnung | CIN | Farbe |
|---|---|---|
| Pigment White 18 | 77713 | weiß |
| Manganammoniumdiphosphat | 77742 | violett |
| Manganphosphat; $Mn_3(PO_4)_2 \cdot 7\ H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzen, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen:

2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfonsäure, Calciumsalz der 2-Hydroxy-1,2'-azonaphthalin-1'-sulfonsäure, Calcium- und Barium salze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfonsäure, Aluminiumsalz der 1-(4-Sulfo-1-naphthylazo)-2-naphthol-3,6-disulfonsäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-(big blank)pyrazolon-3-carbonsäure, Aluminium- und Zirkoniumsalze von 4,5-Dibrom-fluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfonsäure, Aluminiumsalz der Indigo-disulfonsäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, ß-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

    1. "Fischsilber" (Guanin/Hypoxanthin-Mischkristalle aus Fischschuppen) und

    2. "Perlmutt" (vermahlene Muschelschalen)

2. Monokristalline Perlglanzpigmente wie z. B. Bismuthoxychlorid
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung/Schichtdicke | Farbe |
|---|---|---|
| **Silberweiße Perlglanzpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |

(fortgesetzt)

| Gruppe | Belegung/Schichtdicke | Farbe |
|---|---|---|
| | $TiO_2$: 120-160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente , die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30 Gew.%, vorzugsweise von 0,5 bis 15 Gew.%, insbesondere von 1,0 bis 10 Gew.% gewählt, jeweils bezogen auf das Gesamtgewicht der Zubereitungen.

Alle Verbindungen oder Komponenten, die in den Zubereitungen verwendet werden können, sind entweder bekannt und käuflich erwerbbar oder können nach bekannten Verfahren synthetisiert werden.

Eine oder mehrere Verbindungen der Formel Ia-h, II bzw. III können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen mit einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen mit einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden

Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

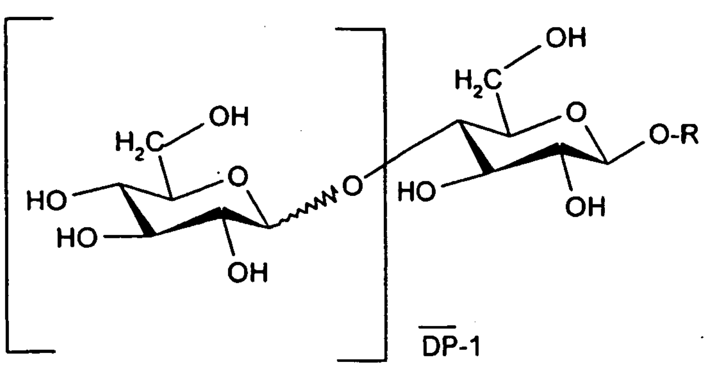

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \Sigma \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

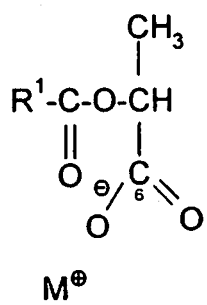

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

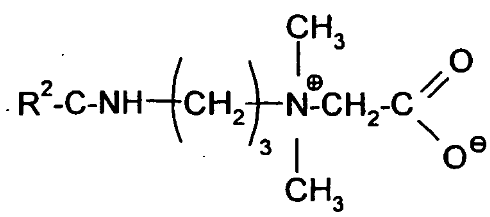

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt,

wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethytenglycol(17)-stearylether (Steareth-17),Polyethylenglycol(18) stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20) isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(1 4)oleylether (Oleth-14), Polyethylenglycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,

Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth-4-sulfat vorteilhaft verwendet werden. Als ethoxyliertes Cholesterinderivat kann vorteilhaft Polyethylenglycol(30)cholesterylether verwendet werden. Auch Polyethylenglycol(25) sojasterol hat sich bewährt. Als ethoxylierte Triglyceride können vorteilhaft die Polyethylenglycol(60) Evening Primrose Glyceride verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol-(20)glyceryloleat, Polyethylenglycol(20)glycerylisostearat, Polyethylenglycol(18)glyceryloleatcocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -Milch vorliegt und außer der oder den Verbindungen der Formel Ia-h, II bzw. Formel III beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann außer der oder den Verbindungen der Formel Ia-h, II bzw. III verschiedene, in diesem Mitteltyp verwendete Adjuvantien enthalten, wie grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Weitere Gegenstände der vorliegenden Erfindung sind ein Verfahren zur Herstellung einer Zubereitung, welches dadurch gekennzeichnet ist, dass mindestens eine Verbindung der Formel Ia-h, II bzw. III mit Resten wie oben beschrieben mit einem kosmetisch oder dermatologisch oder für Nahrungsmittel geeigneten Träger vermischt wird, und die Verwendung einer Verbindung der Formel Ia-h, II bzw. III zur Herstellung einer Zubereitung.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel Ia-h, II bzw. III in dem Träger zur Folge haben.

Es wurde auch festgestellt, dass Verbindungen der Formel Ia-h, II bzw. III stabilisierend auf die Zubereitung wirken können. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Die positiven Wirkungen von Verbindungen der Formel Ia-h, II bzw. III ergeben deren besondere Eignung zur Verwendung in kosmetischen oder pharmazeutischen Zubereitungen.

Ebenso positiv sind die Eigenschaften von Verbindungen mit der Formel Ia-h, II bzw. III zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel Ia-h, II bzw. III angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Weitere Gegenstände der vorliegenden Erfindung sind dementsprechend die Verwendung einer Verbindung nach Formel Ia-h, II bzw. Formel III als Nahrungsmittelzusatz für die human- oder Tierernährung sowie Zubereitungen, die Nahrungsmittel oder Nahrungsergänzungsmittel sind und entsprechende Träger enthalten.

Nahrungsmittel, die nach der vorliegenden Erfindung mit einer oder mehreren Verbindungen der Formel Ia-h, II bzw. III angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit einer oder mehreren Verbindungen der Formel Ia-h, II bzw. III angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel Ia-h, II bzw. III angereichert zu werden, beispielsweise Multivitaminpräparate, Mineralstoffmischungen oder gezuckerter Saft. Als weitere Beispiele für Nahrungsmittel, die nach der vorwiegenden Erfindung mit einer oder mehreren Verbindungen der Formel Ia-h, II bzw. Formel IIII angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel Ia-h, II bzw. III angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel Ia-h, II bzw. Formel III angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel Ia-h, II bzw. Formel III angereicherten erfindungsgemäßen Nahrungsmittel können mit Hilfe von Techniken hergestellt werden, die dem Fachmann wohl bekannt sind.

Desweiteren besitzen Verbindungen der Formel Ia-h, II oder III nur eine schwach ausgeprägte Eigenfarbe. Die schwach ausgeprägte Eigenfarbe ist z.B. dann von großem Vorteil, wenn in den Produkten eine Eigenfarbe der Inhaltsstoffe aus ästhetischen Gründen unerwünscht ist.

Der Anteil der Verbindungen der Formel Ia-h, II bzw. III in der Zubereitung beträgt vorzugsweise von 0,01 bis 20 Gew.%, besonders bevorzugt von 0,05 bis 10 Gew.% und insbesondere bevorzugt von 0,1 bis 5 Gew.% bezogen auf die gesamte Zubereitung. Ganz außerordentlich bevorzugt beträgt der Anteil der Verbindungen der Formel Ia-h, II bzw. III in der Zubereitung von 0,1 bis 2 Gew.% bezogen auf die gesamte Zubereitung.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die folgenden Beispiele sollen die vorliegende Erfindung verdeutlichen. Sie sind jedoch keinesfalls als limitierend zu betrachten. Alle Verbindungen oder Komponenten, die in den Zubereitungen verwendet werden können, sind entweder bekannt und käuflich erhältlich oder können nach bekannten Methoden synthetisiert werden. Die INCI-Namen der verwendeten Rohstoffe sind wie folgt (die INCI-Namen werden definitionsgemäß in englischer Sprache angegeben):

Ein weiterer Gegenstand der Erfindung ist die Verwendung mindestens einer Verbindung gemäß den Formel Ia-h, II oder III oder einer Zubereitung zur Pflege, Konservierung oder Verbesserung des allgemeinen Hautzustandes der Haut oder Haare.

Besonders bevorzugt ist die Verwendung einer Verbindung nach den Formeln Ia-h, II oder III als hautbindende UV-Filter.

Außerdem bevorzugt ist die Verwendung einer Verbindung nach den Formeln Ia-h, II oder III als hautbräunende Wirkstoffe.

**Beispiele**

**<u>Beispiel 1:</u> Synthese von DHA-Hydoxymethylbenzylidencampfer**

**1.1. Synthese von 2-Methyl-2-methoxy-1,3-dioxan-5-on (nach JACS, (1997) 119, 2795-2803)**

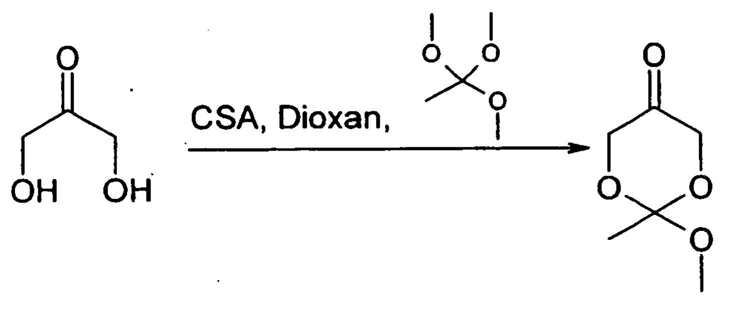


10 g Dihydroxyaceton (111 mmol) und 129 mg (+/-)-Campher-10-sulfonsäure (0,55 mmol, 0,01 Äq.) werden in 500 ml Dioxan gelöst. Es wird 10 min bei 60°C gerührt und dann 69,5 ml Trimethylorthoacetat (555 mmol, 10 Äq.) zugetropft. Nach 16 Std. Reaktionszeit bei 60°C wird das Lösungsmittel im Vakuum entfernt. Der Rückstand wird durch eine Vakuum-Destillation gereinigt. Man erhält 10,71 g (66%) 2-Methyl-2-methoxy-1,3-dioxan-5-on als klares farbloses Öl.

1.2. Synthese von 3-(4-Diethoxymethyl-benzyliden)-campher:

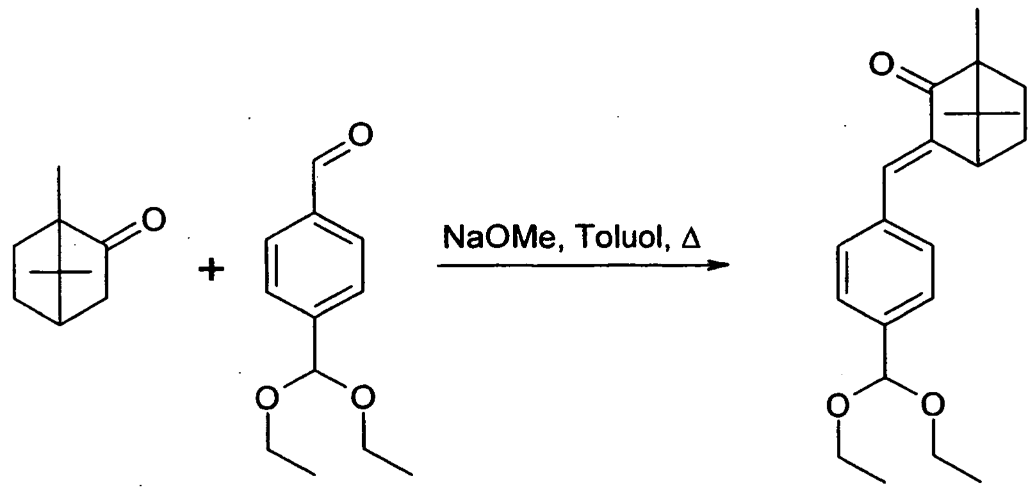


20 g DL-Campher (131 mmol) werden in 640 ml Toluol gelöst und 34,49 g Natriummethanolat (656,9 mmol, 5 Äq.) zugegeben. Es wird für 1 Std. zum Rückfluss erhitzt. Nach Abkühlen auf Raumtemperatur wird das in 60 ml Toluol gelöste Terephthalaldehyd-paramonoethylacetal (26,3 ml, 131 mmol, 1 Äq.) langsam zugetropft. Anschießend wird erneut für 30 min zum Rückfluss erhitzt. Nach Abkühlen wird nach Zugabe von 300 ml gesättigter NaCl-Lösung extrahiert. Nach Trocknen über Natriumsulfat wird das Lösungsmittel im Vakuum entfernt. Man erhält ca. 40 g 3-(4-Diethoxymethyl-benzyliden)-campher als gelbes Öl, das ohne weitere Aufreinigung weiter umgesetzt wird.

1.3. Synthese von 3-(4-Formyl-benzyliden)-campher:

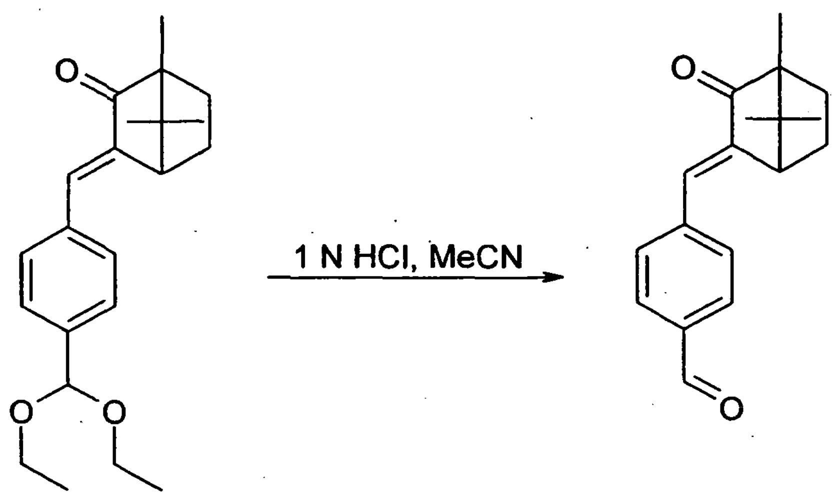


Der 3-(4-Diethoxymethyl-benzyliden)-campher (40 g) wird in 120 ml Acetonitril gelöst und bei 0°C 13 ml 1 N HCl (2,1 Äq.) zugetropft. Nach 1 Std. werden 200 ml Wasser zugegeben und mit 3 x 200 ml Essigsäureethylester extrahiert. Nach Trocknen über Natriumsulfat wird das Lösungsmittel im Vakuum entfernt. Durch mehrmaliges Kristallisieren des Rohprodukts aus n-Heptan in der Siedehitze erhält man 11,14 g (35,3%) 3-(4-Formyl-benzyliden)-campher als weißes Kristallpulver.

**1.4. Umsetzung von 2-Methyl-2-methoxy-1,3-dioxan-5-on mit 3-(4-Formyl-benzyliden)-campher zum Aldoladdukt**

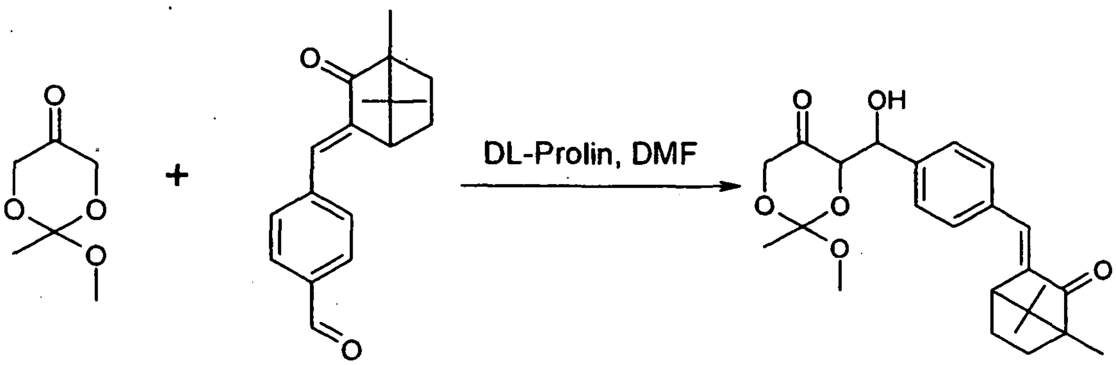


Es werden 817 mg 2-Methyl-2-methoxy-1,3-dioxan-5-on (5,59 mmol, 1.5Äq.) mit 129 mg DL-Prolin (0,3Äq.) in 5 ml DMF gelöst. Nach 30 min Rühren bei Raumtemperatur werden 1 g 3-(4-Formyl-benzyliden)-campher (1 Äq.) zugegeben und für 5 Tage bei Raumtemperatur gerührt. Es werden 15 ml gesättigte $NH_4Cl$-Lösung zugegeben und nach 30 min die Phasen getrennt. Nach Trocknen über Natriumsulfat wird das Lösungsmittel im Vakuum entfernt. Durch saure Hydrolyse des Orthoacetats bzw. Aldoladduktes entsteht Zielstruktur DHA-Hydroxymethylbenzylidencampher.

**1.5. Synthese von 2-Methoxy-4,6-bis-[1-(4-methoxy-phenyl)-meth-(Z)-yliden]-2-methyl-1,3-dioxinan-5-on**

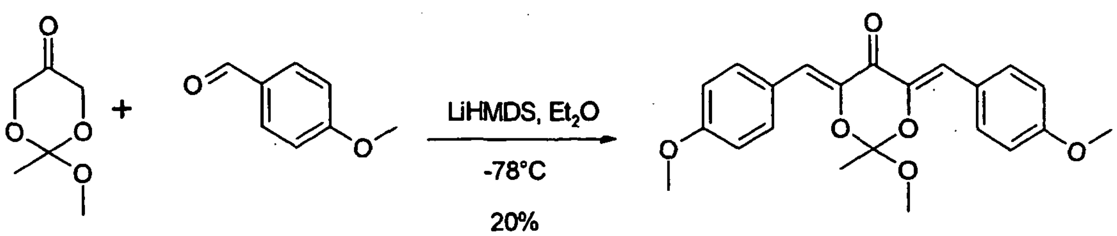


Es werden 500 mg DHA-ortho-methyl-acetat (3,42 mmol) in 5 ml abs. Ether unter inerten Arbeitsbedingungen gelöst und bei -78°C 3,8 ml LiHMDS-Lösung (1 M in THF; 1,1 Äq., 3,76 mmol) zugetropft. Der in 5 ml abs. Ether gelöste Anisaldehyd (466 mg, 3,42 mmol, 1 Äq.) wird anschließend bei -78°C zugetropft. Es wird auf Raumtemperatur erwärmt und nach 96 h durch Gießen auf 10 ml ges. $NH_4Cl$-Lösung aufgearbeitet. Es wird extrahiert, über Natriumsulfat getrocknet und das Lösungsmittel im Vakuum entfernt. Nach chromatographischer Reinigung erhält man 260 mg Produkt (40%) als gelben Feststoff.

**Beispiel 2: Synthese von DHA-Methylbenzylidencampher**

**2.1. Synthese von 4'-Brommethyl-3-benzylidencampfer (nach DE 3445365)**

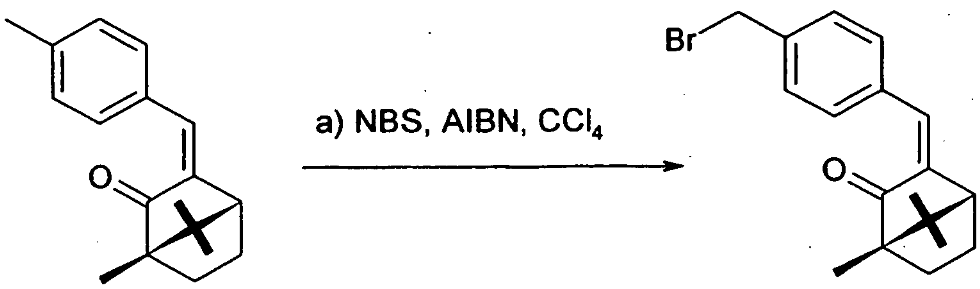


33,75 g (132,7 mmol) Methylbenzylidencampher (z.B. Eusolex 6300) werden in 485 ml $CCl_4$ gelöst, 291 mg AIBN (Kat.) und 24,25 g (136,2 mmol) N-Bromsuccinimid zugefügt und für 1 Std. unter Rückfluss erhitzt. Das entstandene Succinimid wird abfiltriert und das Lösungsmittel im Vakuum entfernt. Anschließend wird aus iso-Propanol und n-Heptan umkristallisiert. Es werden 32,25 g (101, 5 mmol; 76,5%) 4'-Brommethyl-3-benzylidencampher als UV-Filter-Edukt isoliert.

**2.2. Synthese von 2,2-Dimethyl-1,3-dioxon-5-on**

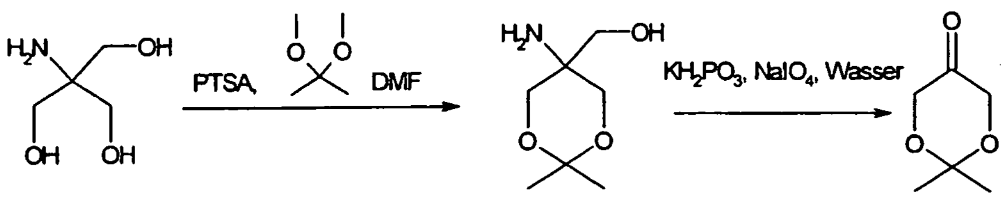


83 g (526,3 mmol Tris(hydroxymethyl)-aminomethanhydrochlorid werden in 327 ml DMF gelöst und mit 5 g (26.3 mmol) para-Toluolsulfonsäure (PTSA; Kat.) und 104 ml (838,8 mmol) 2,2-Dimethoxypropan bei Raumtemp. Umgesetzt. Das Lösungsmittel wird im Vakuum entfernt und der Rückstand in 1700 ml Essigsäureethylester aufgenommen. Nach Zugabe von 73 ml Triethylamin wird der entstandene Feststoff abfiltriert. Das Lösungsmittel wird im Vakuum entfernt und der Rückstand aus MTBE umkristallisiert. Man erhält 54,9 g (64,7%) (5-Amino-2,2-dimethyl-1,3-dioxinan-5-yl)-methanol als weißen Feststoff. Dieser wird dann in 1l Wasser gelöst und mit 46,3 g kaliumdihydrogenphosphat (1Äq.) versetzt und bei 0°C das in 1,1 l Wasser gelöste Natriumperiodat (72,8 g) langsam zugetropft. Nach 16 h Reaktion bei RT werden 53,8 g Natriumthiosulfat zugegeben und die wässrige Phase so lange mit Dichlormethan extrahiert, bis sie produktfrei ist. Nach Vakuum-Destillation erhält man 30,8 g (69,5%) 2,2-Dimethyl-1,3-dioxon-1,3. (siehe Tetrahedron, 1989, 45, 3, 687-694, D. Hoppe)

**2.3 Synthese von DHA-Methylenbenzylidencampher**

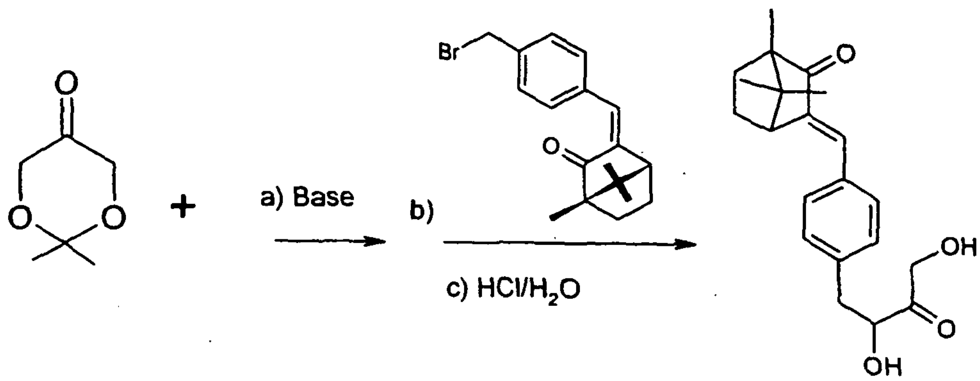


3,3 g (15,4 mmol) 2,2-Dimethyl-1,3-dioxon-5-cycloheylimin wird in 34 ml abs. THF gelöst und bei -78°C mit 16,1 ml LiHMDS-Lösung (1 mol/l in THF, 16,1 mmol) deprotoniert. Nach 15 Min. werden 5, 6 g (16,9 mml) 4'-Brommethyl-3-benzylidencampher, gelöst in 5 ml abs. THF zugetropft. Nach Reaktion für 16 h wird durch Zugabe von ges. $NH_4Cl$-Lösung aufgearbeitet.

**<u>Beispiel 3:</u> Synthese von DHA-Methythydroxyphenylbenzotriazol**

**3.1. Synthese von o-tert. Butyldimethylsilylbenzotriazolbromid**

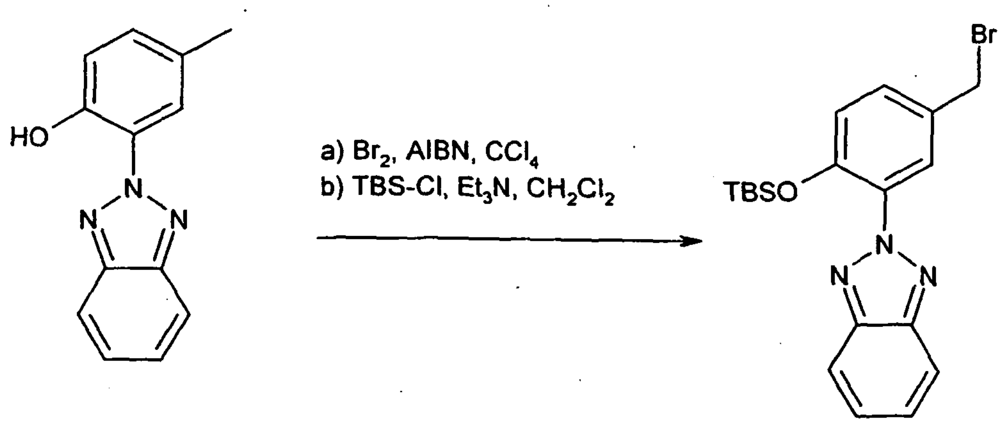


5 g (22,3 mmol) 2-(2H-Benzotriazole-2-yl)-4-methylphenol wird in 150 ml $CCl_4$ gelöst sowie 100 mg AIBN (Kat.) zugefügt. Das in 75 ml $CCl_4$ gelöste Brom (4,15g, 30 mmol) wird zugetropft und dann für 5 h auf 47 °C erhitzt.

Das Lösungsmittel wird im Vakuum entfernt und das Produkt durch Kristallisation aus Aceton erhalten. Man erhält 5,5 g (80%) 2-2(2H-Benzotriazole-2-yl)-4-bromomethylphenol. (siehe US 6,284,895) Im Anschluss wird das bromierte Benzotriazol in 200 ml Dichlormethan gelöst und mit 3,8 ml (27,1 mmol) Triethylamin und 3 g TBS-Chlorid (19,9 mmol) am Phenol silyliert. Es entsteht 6,05 g 2-[5-Bromomethyl-2-(tert.-butyl-dimethyl-silanyloxyrphenyl]-2H-benzotriazole (80%).

**3.2. Synthese von DHA-Dibenzoylestercyclohexylimin**

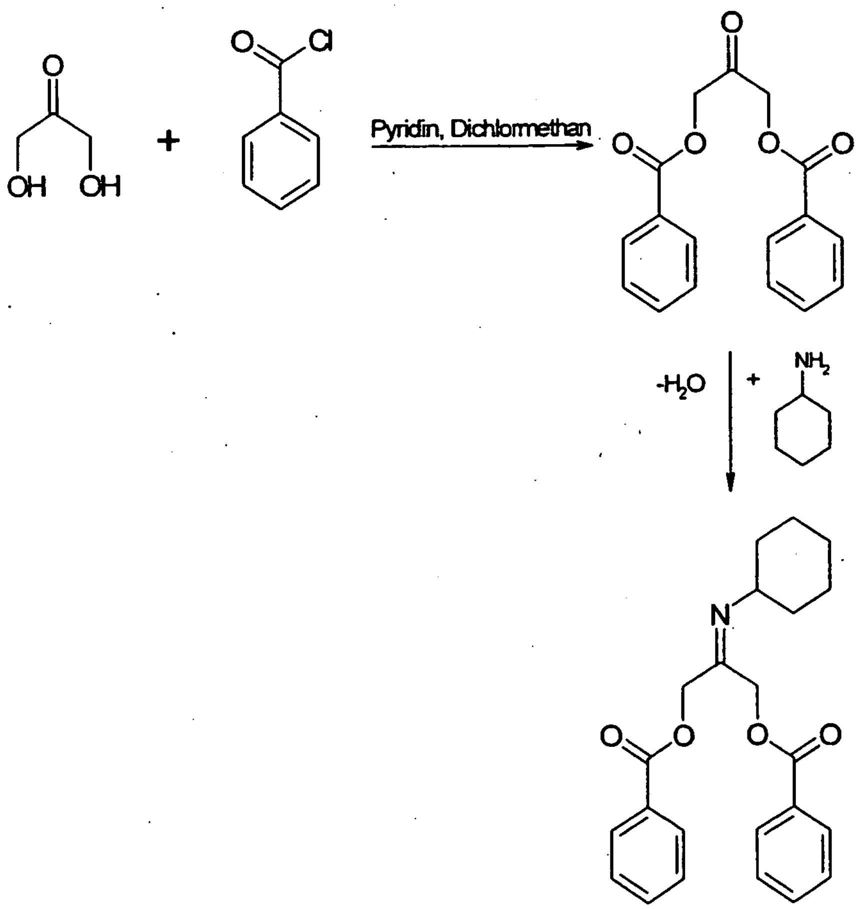


15 g (166,5 mmol) Dihydroxyaceton (dimer) wird 2 h in 150 ml Pyridin monomerisiert. Anschließend erfolgt bei 0° C die Zugabe von 42,6 ml (366 mmol) Benzoylchlorids gelöst in 50 ml Dichlormethan. Nach 16 h Reaktion bei RT wird das Lösungmittel im Vakuum entfernt. Es wird aus Essigsäureethylester und n-Heptan kristallisiert. Man erhält 27.1 g (54,6%) DHA-Dibenzoylester.
Im zweiten Schritt wird der DHA-Dibenzoylester mit 1Äq. Cycloehexylamin und 2 g/mmol 4 A Molekularsieb in 5ml/mmol Benzol zum DHA-dibenzoylestercyclohexylimin umgesetzt.

### 3.3. Synthese von DHA-Methylhydroxyphenylbenzotriazol

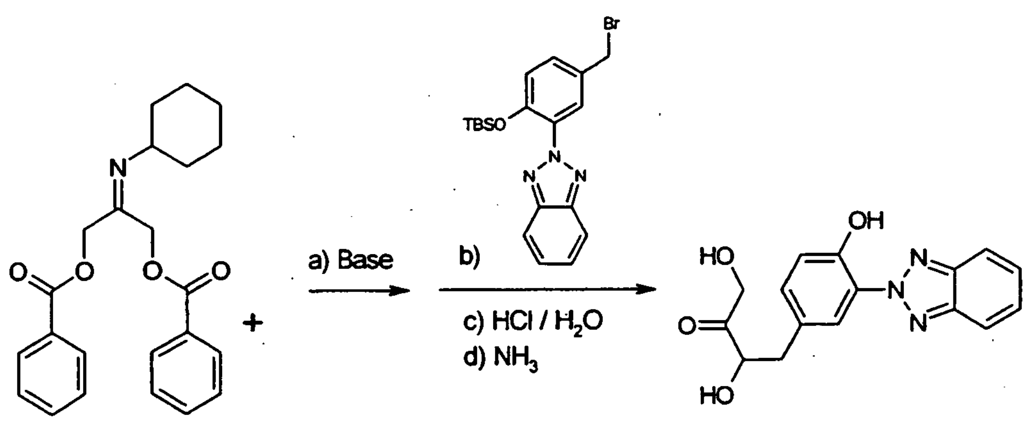

Die Synthese von DHA-Methylhydroxyphenylbenzotriazol erfolgt analog zur Synthese unter Beispiel 2.3.

**Beispiel 4: Lotion (W/O) zum Auftragen auf die Haut**

| | | **Gew.%** |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | Produkt aus einem der Beispiele 1-3 | 0,5 |
| **B** | Glycerin | 5,0 |
| | Magnesiumsulfat-Heptahydrat | 1,0 |
| | Konservierungsmittel | q.s. |
| | Wasser, demineralisiert | ad 100 |

Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:

0,05 % Propyl-4-hydroxybenzoat
0,15 % Methyl-4-hydroxybenzoat

**Beispiel 5: Lotion (W/O) zum Auftragen auf die Haut**

| | | **Gew.%** |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| **B** | Produkt aus einem der Beispiele 1-3 | 1,0 |
| | Glycerin | 5,0 |
| | Magnesiumsulfat-Heptahydrat | 1,0 |
| | Konservierungsmittel | q.s. |
| | Wasser, demineralisiert | ad 100 |

Herstellung

Phase A wird auf 75°C und Phase B auf 80°C erwärmt. Unter Rühren wird Phase B langsam zu Phase A

gegeben. Nach dem Homogenisieren wird unter Rühren abgekühlt. Bei einer Temperatur von 40°C werden Parfümstoffe zugegeben.

Als Konservierungsmittel werden verwendet:

0,05 % Propyl-4-hydroxybenzoat
0,15 % Methyl-4-hydroxybenzoat

**Beispiel 6: Lotion (W/O) zum Auftragen auf die Haut**

| | | Gew.% |
|---|---|---|
| **A** | Produkt aus einem der Beispiele 1-3 | 1,0 |
| | Polyglyceryl-2-Dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | 5,7-Dihydroxy-2-methyl-chromen-4-on | 1,0 |
| | | |
| **B** | **Glycerin** | 5,0 |
| | Magnesiumsulfat-Heptahydrat | 1,0 |
| | Konservierungsmittel | q.s. |
| | Wasser, demineralisiert | ad 100 |

Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:

0,05 % Propyl-4-hydroxybenzoat
0,15 % Methyl-4-hydroxybenzoat

**Beispiel 7: Aus folgenden Komponenten wird eine Creme (O/W), enthaltend Ectoin, hergestellt:**

| | | | Gew.% |
|---|---|---|---|
| **A** | Paraffin, dünnflüssig | (1) | 8,0 |
| | Isopropylmyristat | (1) | 4,0 |
| | Mirasil CM5 | (2) | 3,0 |
| | Stearinsäure | (1) | 3,0 |
| | Arlacel 165 V | (3) | 5,0 |
| | Produkt aus einem der Beispiele 1-3 | | 1,0 |
| | | | |
| **B** | Glycerin (87%) | (1) | 3,0 |
| | Germaben II | (4) | 0,5 |
| | Wasser, demineralisiert | | ad 100 |
| | | | |
| **C** | RonaCare™ Ectoin | (1) | 1,0 |

Herstellung

Zunächst werden die Phasen A und B getrennt auf 75˚C erwärmt. Danach wird Phase A unter Rühren langsam zu Phase B gegeben und solange gerührt, bis eine homogene Mischung entsteht. Nach Homogenisierung der Emulsion wird unter Rühren auf 30˚C abgekühlt. Anschließend wird auf 35˚C erwärmt, die Phase C zugegeben und bis zur Homogenität gerührt.

Bezugsquellen

(1) Merck KGaA
(2) Rhodia
(3) Uniqema
(4) ISP

**Beispiel 8: Topische Zusammensetzung als W/O-Emulsion**

| | | | Gew.% |
|---|---|---|---|
| **A** | Isolan PDI | (2) | 3,0 |
| | Paraffinöl, fl. | (1) | 17,0 |
| | Isopropylmyristat | | 5,0 |
| | Bienenwachs | | 0,2 |
| | Cutina HR | (2) | 0,3 |
| | Produkt aus einem der Beispiele 1-3 | | 1,0 |
| **B** | Wasser, demineralisiert | | ad 100 |
| | Glycerin (87%) | | 4,0 |
| | Magnesiumsulfat | | 1,0 |
| | Germaben II-E | (3) | 1,0 |
| **C** | RonaCare™ LPO | (1) | 2,0 |

Herstellung

Die Phasen A und B werden auf 75˚C erwärmt. Phase B wird unter Rühren zu Phase A gegeben. Anschließend wird das Gemisch bei 9000upm 2 Min. mit dem Turrax homogenisiert. Das erhaltene Gemisch wird auf 30 bis 35˚C abgekühlt, und C wird eingerührt.

Bezugsquellen

(1) Merck KGaA
(2) Goldschmidt AG
(3) ISP

**Beispiel 9: Zubereitungen**

Im folgenden werden beispielhaft Rezepturen für kosmetische Zubereitungen angegeben, die Verbindungen ausgewählt aus den Beispielen 1 bis 4 enthalten. Die Bezeichnung der Verbindungen erfolgt dabei entsprechend den Angaben in der Beschreibung. Im übrigen sind die INCI-Bezeichnungen der handelsüblichen Verbindungen angegeben.
UV-Pearl , OMC steht für die Zubereitung mit der INCI-Bezeichnung: Water (for EU: Aqua), Ethylhexyl Methoxycinnamate, Silica, PVP, Chlorphenesin, BHT; diese Zubereitung ist im Handel unter der Bezeichnung Eusolex®UV Pearl™OMC von der Merck KGaA, Darmstadt erhältlich.

Die anderen in den Tabellen angegebenen UV-Pearl sind jeweils analog zusammengesetzt, wobei OMC gegen die angegebenen UV-Filter ausgetauscht wurde.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | | 2 | 5 | | | | | | | 3 |
| 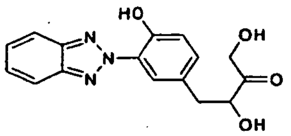 | 5 | 3 | 2 | 1 | 2 | | | | 1 | 1 |
| 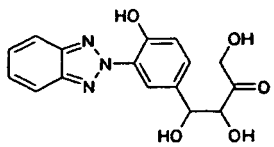 | | | | | | 1 | 2 | 1 | | |
| Zinc oxide | | | | | | | | 5 | 2 | |
| UV-Pearl, OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 . | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |
| | 1-11 | 1-12 | 1-13 | 1-14 | 1-15 | 1-16 | 1-17 | 1-18 | | |
| Titanium dioxide | 3 | | 2 | | 3 | | 2 | 5 | | |
| Benzylidene malonate polysiloxane | | 1 | 0,5 | | | | | | | |
| Methylene Bis-Benztriazolyl Tetramethylbutylphenol | 1 | 1 | 0,5 | | | | | | | |

(fortgesetzt)

| | 1-11 | 1-12 | 1-13 | 1-14 | 1-15 | 1-16 | 1-17 | 1-18 | | |
|---|---|---|---|---|---|---|---|---|---|---|
| 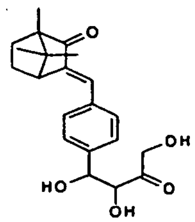 | 5 | 3 | 2 | 5 | 1 | 3 | 7 | 2 | | |
| Polyglyceryl-3-Dimerate | 3 | 3 | 3 | 3 | | | | | | |
| Cera Alba | 0,3 | 0,3 | 0,3 | 0,3 | 2 | 2 | 2 | 2 | | |
| Hydrogenated Castor Oil | 0,2 | 0,2 | 0,2 | 0,2 | | | | | | |
| Paraffinium Liquidum | 7 | 7 | 7 | 7 | | | | | | |
| Caprylic/Capric Triglyceride | 7 | 7 | 7 | 7 | | | | | | |
| Hexyl Laurate | 4 | 4 | 4 | 4 | | | | | | |
| PVP/Eicosene Copolymer | 2 | 2 | 2 | 2 | | | | | | |
| Propylene Glycol | 4 | 4 | 4 | 4 | | | | | | |
| Magnesium Sulfate | 0,6 | 0,6 | 0,6 | 0,6 | | | | | | |
| Tocopherol | 0,5 | 0,5 | 0,5 | 0,5 | | | | | | |
| Tocopheryl Acetate | 0,5 | 0,5 | 0,5 | 0,5 | 1 | 1 | 1 | 1 | | |
| Cyclomethicone | 0,5 | 0,5 | 0,5 | 0,5 | | | | | | |
| Propylparabene | 0,05 | 0,05 | 0,05 | 0,05 | 0,05 | 0,05 | 0,05 | 0,05 | | |
| Methylparabene | 0,15 | 0,15 | 0,15 | 0,15 | 0,15 | 0,15 | 0,15 | 0,15 | | |
| Dicocoyl Pentyerythrityl Citrate (and) Sorbitan Sesquioleate (and) Cera Alba (and) Aluminium Stearate | | | | | 6 | 6 | 6 | 6 | | |
| PEG-7 Hydrogenated Castor Oil | | | | | 1 | 1 | 1 | 1 | | |
| Zinc Stearate | | | | | 2 | 2 | 2 | 2 | | |
| Oleyl Erucate | | | | | 6 | 6 | 6 | 6 | | |
| Decyl Oleate | | | | | 6 | 6 | 6 | 6 | | |
| Dimethicone | | | | | 5 | 5 | 5 | 5 | | |
| Tromethamine | | | | | 1 | 1 | 1 | 1 | | |
| Glycerin | | | | | 5 | 5 | 5 | 5 | | |
| Allantoin | | | | | 0,2 | 0,2 | 0,2 | 0,2 | | |
| Water | ad 100 | ad 100 | ad 100 | ad 100 | ad 100 | ad 100 | ad 100 | ad 100 | | |

**Patentansprüche**

1. Verbindung nach Formel II

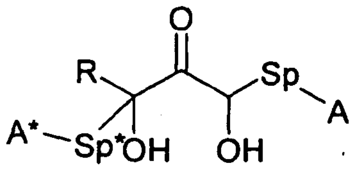

II

worin

- R steht für H oder $(CHOH)_nCH_2OH$ (n= 0, 1 oder 2),
- Sp, Sp* stehen für -CH-, -CHOH-, $-(CH_2)_n-$, $-(CH=CH)_n-$, $-(CH=CH-CH_2)_n-$, $-(CH_2)_n-X-(CH_2)_o-$, $-(CH_2-X)_n-(CH_2)_o-$, $-(CH_2)_n-C(=X)-(CH_2)_o-X-(CH_2)_p-$, $-(CH_2)_n-X-(CH_2)_o-C(=X)-(CH_2)_p-$, $-(CH_2)_n-C(=X)-(CH_2)_o-$, und ist über eine Einfach- oder Doppelbindung an das in Richtung der Carbonylgruppe benachbarte C-Atom gebunden.
- n steht für eine ganze Zahl unabhängig voneinander ausgewählt aus dem Bereich beginnend mit 0 und endend mit 20,
- X steht für O, N oder S,
- A, A* stehen unabhängig voneinander für einen Substituenten der UV-Strahlung absorbiert und ein konjugiertes $\pi$-Elektronensystem von mindestens $4\pi$-Elektronen aufweisen,
- wobei A und A* für gleiche oder verschiedene Reste stehen können.

**2.** Verbindung nach Anspruch 1, **dadurch gekennzeichnet, dass** R steht für H.

**3.** Verbindung nach Formel II nach mindestens einem der Ansprüche 1 oder 2, **dadurch gekennzeichnet, dass** es sich um eine Verbindung aus der folgenden Gruppe handelt:

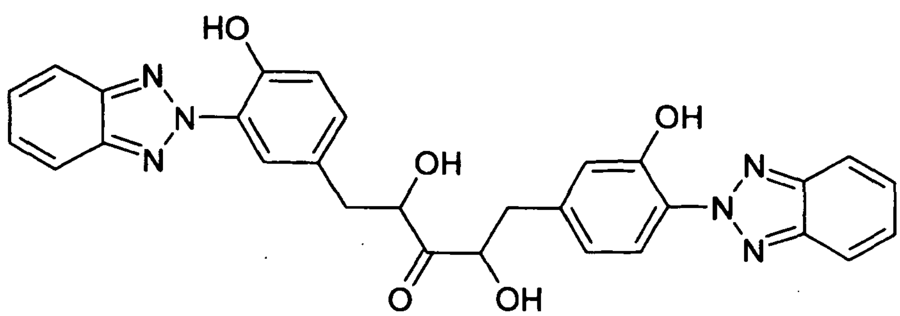

IIa

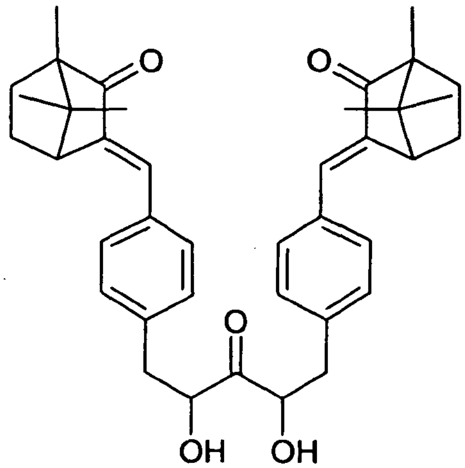

IIb

4. Verbindung **dadurch gekennzeichnet, dass** es sich um eine Verbindung aus der folgenden Gruppe handelt:

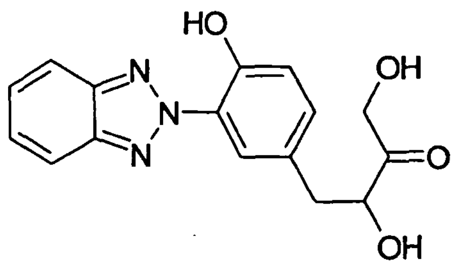

Ia

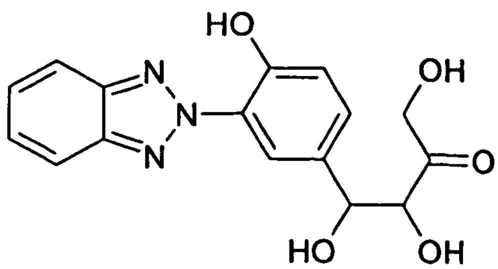

Ib

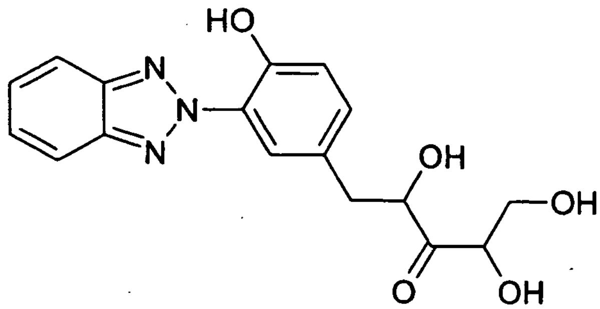

Ic

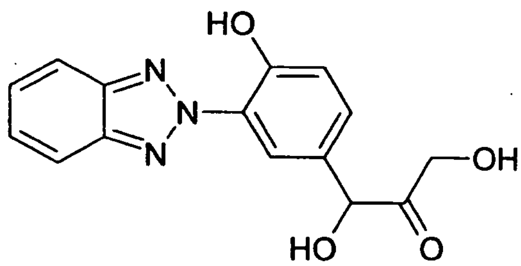

Id

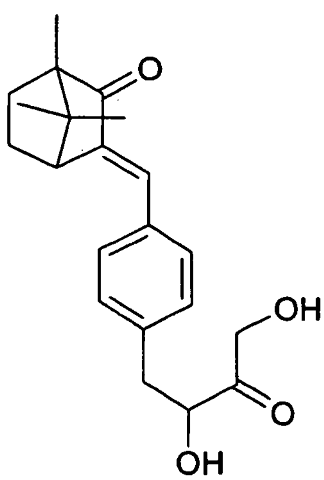

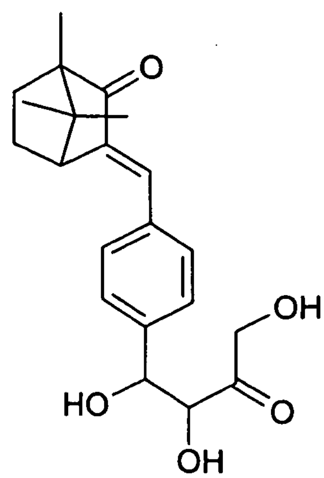

Ie / If

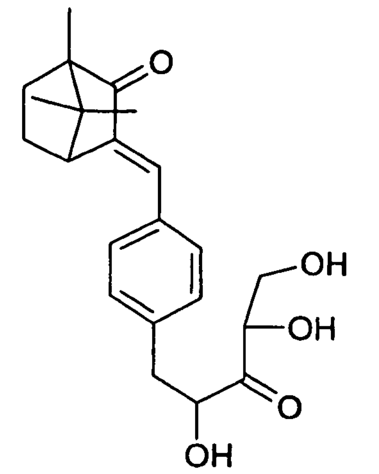

Ig

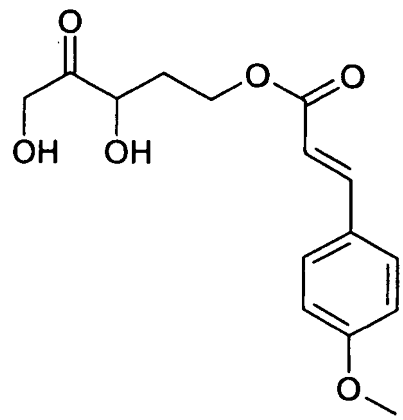

Ih

**5.** Verbindung nach Formel III

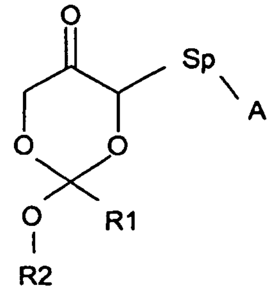

III

worin

- R1, R2 gleich oder verschieden sind und H, Alkyl, Phenyl oder Aryl bedeuten,

  - Sp steht für -CH-, -CHOH-, $-(CH_2)_n-$, $-(CH=CH)_n-$, $-(CH=CH-CH_2)_n-$, $-(CH_2)_n-X-(CH_2)_o-$, $-(CH_2-X)_n-(CH_2)_o-$, $-(CH_2)_n-C(=X)-(CH_2)_o-X-(CH_2)_p-$, $-(CH_2)_n-X-(CH_2)_o-C(=X)-(CH_2)_p-$, $-(CH_2)_n-C$

$(=X)-(CH_2)_o-$, und ist über eine Einfach- oder Doppelbindung an das in Richtung der Carbonylgruppe benachbarte C-Atom gebunden,
- n, p, o stehen für eine ganze Zahl, unabhängig voneinander ausgewählt aus dem Bereich beginnend mit 0 und endend mit 20,
- X steht für O, N oder S,
- A steht unabhängig voneinander für einen Substituenten der UV-Strahlung absorbiert und ein konjugiertes $\pi$-Elektronensystem von mindestens $4\pi$-Elektronen aufweist.

- wobei A für gleiche oder verschiedene Reste stehen kann.

6. Verbindung nach Anspruch 5, **dadurch gekennzeichnet, dass** es sich um einen Dihydroxyaceton-Orthoester aus der folgenden Gruppe handelt:

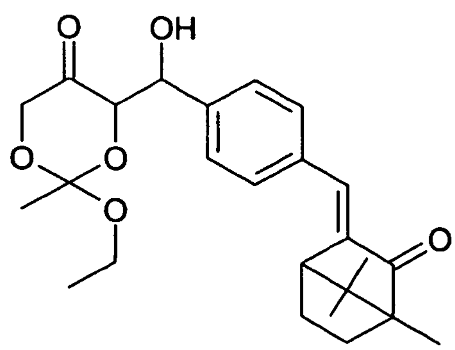

IIIa

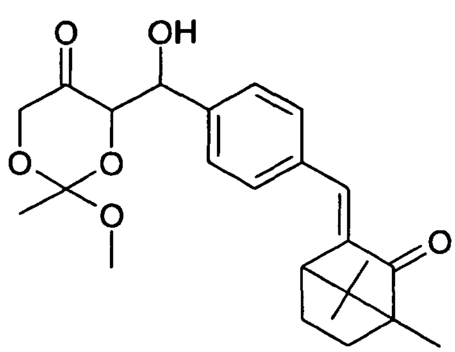

IIIb

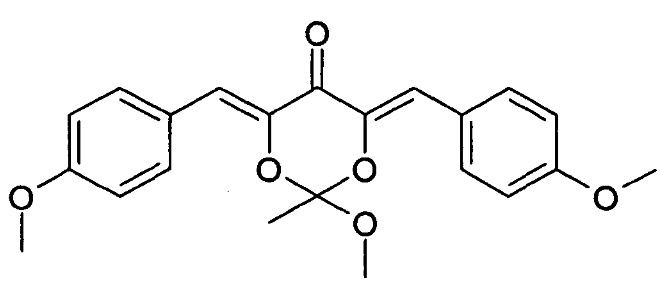

IIIc

7. Verfahren zur Herstellung von Verbindungen der Formeln Ia-h erhalten durch Umsetzung einer Verbindung der Formel

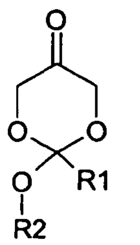

worin

- R1, R2 gleich oder verschieden sind und H, Methyl, Ethyl, Alkyl, Phenyl, Aryl bedeuten
- wobei die Verbindung 2-Methyl-2-methoxy-1,3-dioxan-5-on ausgeschlossen ist,

mit

a) einer Base wie Lithiumdiisopropylamid oder Lithiumhexamethyldisilazan,
b) mit ortho-tert-Butyldimethylsilylbenzotriazolbromid

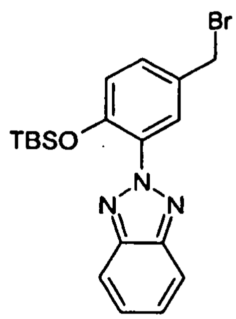

oder -aldehyd, Zimtsäurechlorethlyester, Methylbenzylidencampherbromid oder -aldehyd und
c) HO/$H_2O$.

**8.** Zubereitung enthaltend einen Träger, **dadurch gekennzeichnet, dass** die Zubereitung 0.01 bis 20 Gew.% mindestens einer Verbindung nach den Formeln Ia-h, II oder III gemäß Anspruch 1 bis 6 enthält.

**9.** Zubereitung nach Anspruch 8, **dadurch gekennzeichnet, dass** eine oder mehrere Verbindungen der Formel Ia-h, II oder III in Mengen von 0.05 bis 10 Gew.%, vorzugsweise 0.1 bis 5 Gew.% in der Zubereitung enthalten sind.

**10.** Zubereitung nach mindestens einem der vorstehenden Ansprüche, **dadurch gekennzeichnet, dass** die Zubereitung ein oder mehrere Antioxidantien und/oder einen oder mehrere weitere UV-Filter enthält.

**11.** Verwendung einer Verbindung gemäß Formeln Ia-h, II oder III oder einer Zubereitung nach mindestens einem der Ansprüche 8 bis 10 zur Prophylaxe gegen zeit- und/oder lichtinduzierte Alterungsprozesse der menschlichen Haut oder menschlicher Haare, insbesondere zur Prophylaxe gegen trockene Haut, Faltenbildung und/oder Pigmentstörungen, und/oder zur Reduktion oder Verhinderung schädigender Effekte von UV-Strahlen auf die Haut.

**12.** Verwendung einer Verbindung gemäß Formel Ia-h, II oder III oder einer Zubereitung nach mindestens einem der Ansprüche 8 bis 10 zur Prophylaxe gegen oder Reduktion von Hautunebenheiten, wie Falten, feinen Linien, rauher oder großporiger Haut.

**13.** Verfahren zur Herstellung einer Zubereitung nach mindestens einem der Ansprüche 8 bis 10, **dadurch gekennzeichnet, dass** mindestens eine Verbindung der Formel Ia-h, II oder III mit Resten wie oben beschrieben mit einem kosmetisch oder dermatologisch oder für Nahrungsmittel geeigneten Träger vermischt wird.

**14.** Verwendung mindestens einer Verbindung der Formeln Ia-h, II oder III zur Herstellung einer Zubereitung nach mindestens einem der Ansprüche 8 bis 10.

**15.** Verwendung mindestens einer Verbindung gemäß Formeln Ia-h, II oder III oder einer Zubereitung nach mindestens einem der Ansprüche 8 bis 10 zur Pflege, Konservierung oder Verbesserung des allgemeinen Zustandes der Haut oder Haare.

**16.** Verwendung einer Verbindung nach den Formeln Ia-h, II oder III gemäß einem der Ansprüche 1 bis 6 als hautbindende UV-Filter.

**17.** Verwendung einer Verbindung nach den Formeln Ia-h, II oder III gemäß einem der Ansprüche 1 bis 6 als hautbräunende Wirkstoffe.

## Claims

**1.** Compound of the formula II

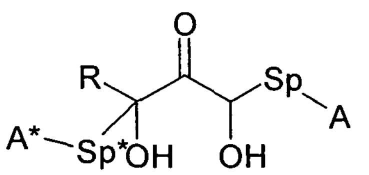

II

in which

- R stands for H or $(CHOH)_nCH_2OH$ (n = 0, 1 or 2),
- Sp, Sp* stand for -CH-, -CHOH-, $-(CH_2)_n-$, $-(CH=CH)_n-$, $-(CH=CH-CH_2)_n-$, $-(CH_2)_n-X-(CH_2)_o-$, $-(CH_2-X)_n-(CH_2)_o-$, $-(CH_2)_n-C(=X)-(CH_2)_o-X-(CH_2)_p-$, $-(CH_2)_n-X-(CH_2)_o-C(=X)-(CH_2)_p-$, $-(CH_2)_n-C(=X)-(CH_2)_o-$, and are bonded via a single or double bond to the C atom which is adjacent in the direction of the carbonyl group,
- n stands for an integer selected, independently of one another, from the range starting with 0 and ending with 20,
- X stands for O, N or S,
- A, A* stand, independently of one another, for a substituent which absorbs UV radiation and have a conjugated $\pi$ electron system of at least 4 $\pi$ electrons,
- where A and A* may stand for identical or different radicals.

**2.** Compound according to Claim 1, **characterised in that** R stands for H.

**3.** Compound of the formula II according to at least one of Claims 1 or 2, **characterised in that** it is a compound from the following group:

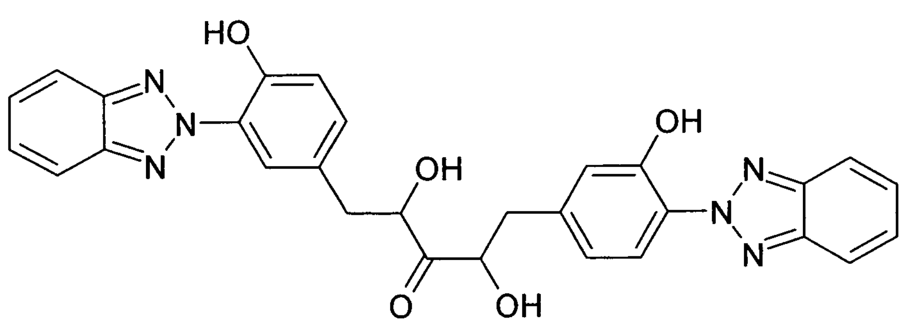

IIa

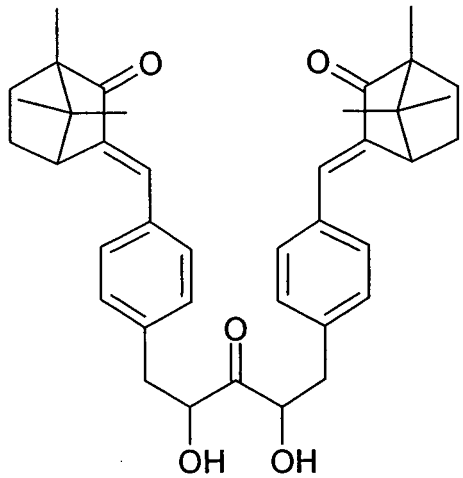

IIb

4. Compound, **characterised in that** it is a compound from the following group:

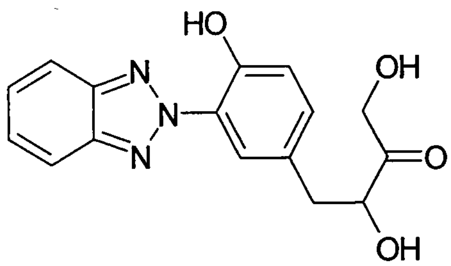

Ia

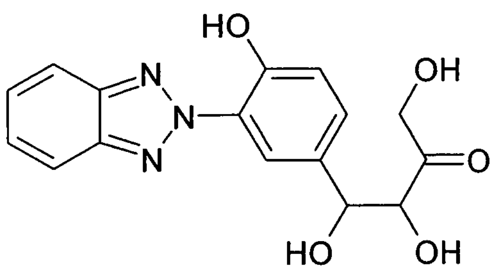

Ib

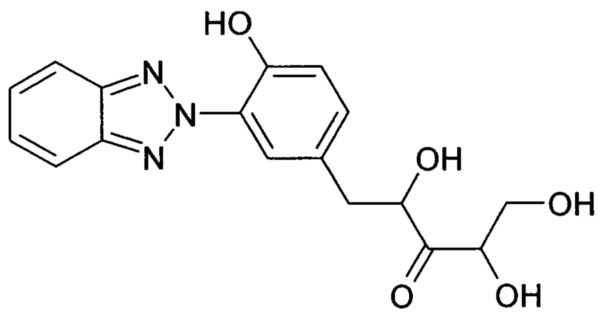

Ic

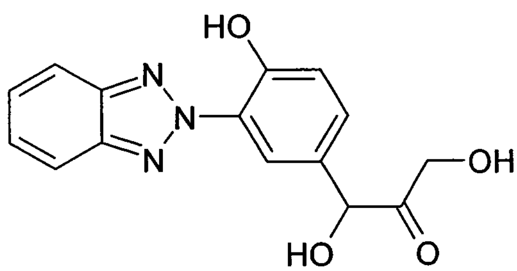

Id

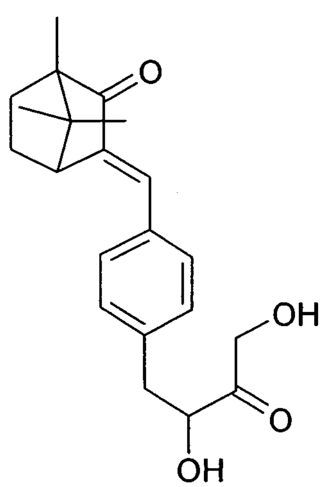

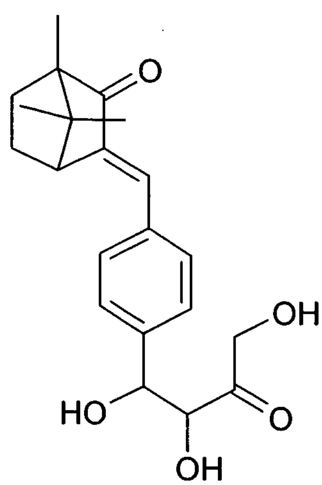

Ie / If

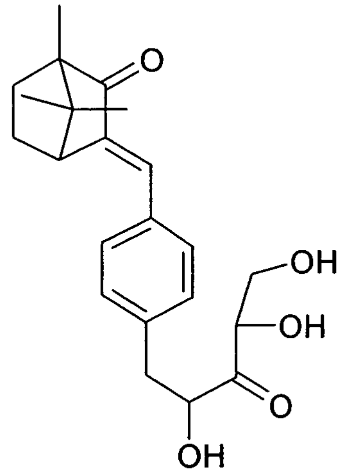

Ig

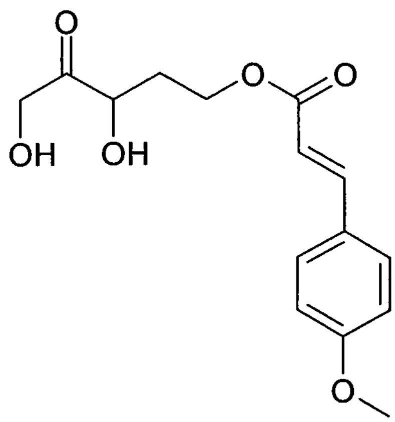

Ih

**5.** Compound of the formula III

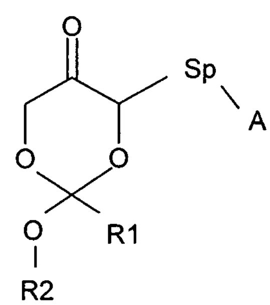

III

in which

- R1, R2 are identical or different and denote H, alkyl, phenyl or aryl,
- Sp stands for -CH-, -CHOH-, $-(CH_2)_n-$, $-(CH=CH)_n-$, $-(CH=CH-CH_2)_n-$, $-(CH_2)_n-X-(CH_2)_o-$, $-(CH_2-X)_n-(CH_2)_o-$, $-(CH_2)_n-C(=X)-(CH_2)_o-X-(CH_2)_p-$, $-(CH_2)_n-X-(CH_2)_o-C(=X)-(CH_2)_p-$, $-(CH_2)_n-C(=X)-(CH_2)_o-$, and is bonded via a single or double bond to the C atom which is adjacent in the direction of the carbonyl group,
- n, p, o stand for an integer selected, independently of one another, from the range starting with 0 and ending

with 20,
- X stands for O, N or S,
- A stands, independently of one another, for a substituent which absorbs UV radiation and has a conjugated $\pi$ electron system of at least 4 $\pi$ electrons,
- where A may stand for identical or different radicals.

**6.** Compound according to Claim 5, **characterised in that** it is a dihydroxyacetone orthoester from the following group:

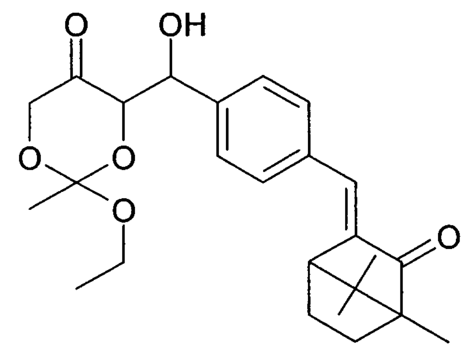

IIIa

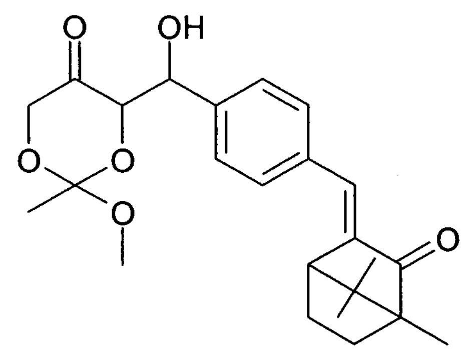

IIIb

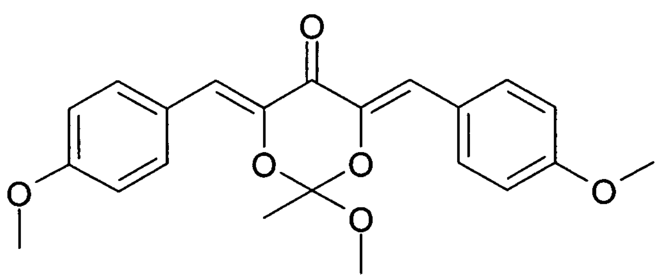

IIIc

**7.** Process for the preparation of compounds of the formulae Ia-h by reaction of a compound of the formula

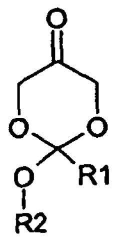

in which

- R1, R2 are identical or different and denote H, methyl, ethyl, alkyl, phenyl, aryl,
- where the compound 2-methyl-2-methoxy-1,3-dioxan-5-one is excluded,

with

a) a base, such as lithium diisopropylamide or lithium hexamethyldisilazane,
b) with ortho-tert-butyldimethylsilylbenzotriazole bromide

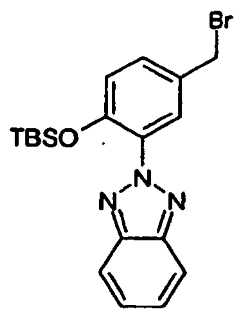

or aldehyde, chloroethyl cinnamate, methylbenzylidenecamphor bromide or aldehyde and
c) $HCl/H_2O$.

8. Composition comprising a vehicle, **characterised in that** the composition comprises 0.01 to 20% by weight of at least one compound of the formulae Ia-h, II or III according to Claims 1 to 6.

9. Composition according to Claim 8, **characterised in that** one or more compounds of the formulae Ia-h, II or III are present in the composition in amounts of 0.05 to 10% by weight, preferably 0.1 to 5% by weight.

10. Composition according to at least one of the preceding claims, **characterised in that** the composition comprises one or more antioxidants and/or one or more further UV filters.

11. Use of a compound of the formula Ia-h, II or III or of a composition according to at least one of Claims 8 to 10 for prophylaxis against time- and/or light-induced ageing processes of the human skin or human hair, in particular for prophylaxis against dry skin, wrinkling and/or pigment defects, and/or for the reduction or prevention of damaging effects of UV rays on the skin.

12. Use of a compound of the formula Ia-h, II or III or of a composition according to at least one of Claims 8 to 10 for prophylaxis against or the reduction of skin irregularities, such as wrinkles, fine lines, rough or large-pored skin.

13. Process for the preparation of a composition according to at least one of Claims 8 to 10, **characterised in that** at least one compound of the formula Ia-h, II or III containing radicals as described above is mixed with a vehicle which is suitable cosmetically or dermatologically or for foods.

14. Use of at least one compound of the formula Ia-h, II or III for the preparation of a composition according to at least one of Claims 8 to 10.

15. Use of at least one compound of the formula Ia-h, II or III or of a composition according to at least one of Claims 8 to 10 for the care, preservation or improvement of the general state of the skin or hair.

16. Use of a compound of the formula Ia-h, II or III according to one of Claims 1 to 6 as skin-binding UV filter.

17. Use of a compound of the formula Ia-h, II or III according to one of Claims 1 to 6 as skin-tanning active compound.

**Revendications**

1. Composé de formule II

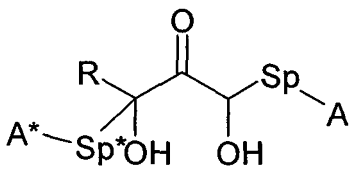

II

dans laquelle

- R représente H ou $(CHOH)_nCH_2OH$ (n = 0, 1 ou 2),
- Sp, Sp* représentent -CH-, -CHOH-, $-(CH_2)_n-$, $-(CH=CH)_n-$, $-(CH=CH-CH_2)_n-$, $-(CH_2)_n-X-(CH_2)_o-$, $-(CH_2-X)_n-(CH_2)_o-$, $-(CH_2)_n-C(=X)-(CH_2)_o-X-(CH_2)_p-$, $-(CH_2)_n-X-(CH_2)_o-C(=X)-(CH_2)_p-$, $-(CH_2)_n-C(=X)-(CH_2)_o-$, et sont liés par l'intermédiaire d'une liaison simple ou double à l'atome de C qui est adjacent en direction du groupement carbonyle,
- n représente un nombre entier choisi, indépendamment l'un de l'autre, dans la plage commençant par 0 et finissant par 20,
- X représente O, N ou S,
- A, A* représentent, indépendamment l'un de l'autre, un substituant qui absorbe le rayonnement UV et possèdent un système d'électrons $\pi$ conjugué d'au moins 4 électrons $\pi$,
- où A et A* peuvent représenter des radicaux identiques ou différents.

2. Composé selon la revendication 1, **caractérisé en ce que** R représente H.

3. Composé de formule II selon au moins l'une des revendications 1 ou 2, **caractérisé en ce qu'**il s'agit d'un composé issu du groupe suivant :

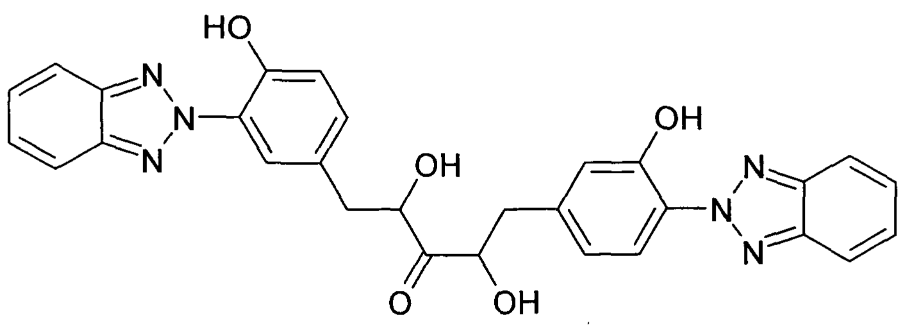

IIa

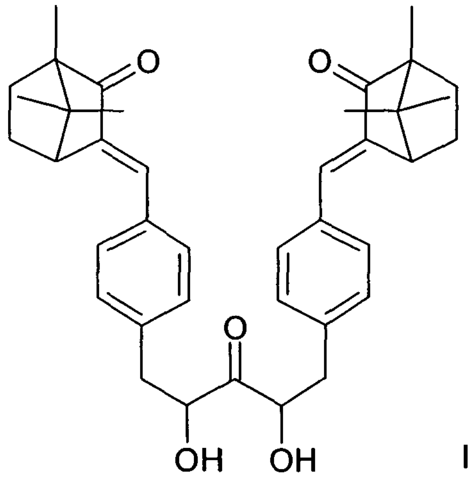

IIb

4. Composé, **caractérisé en ce qu'**il s'agit d'un composé issu du groupe suivant :

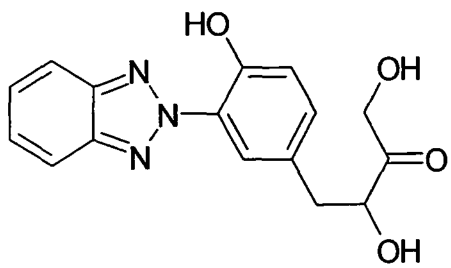

Ia

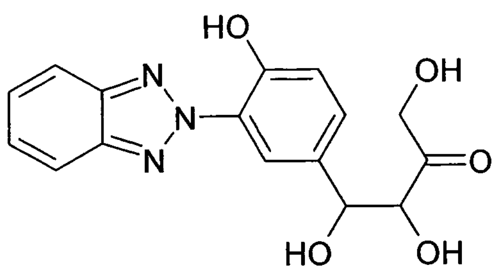

Ib

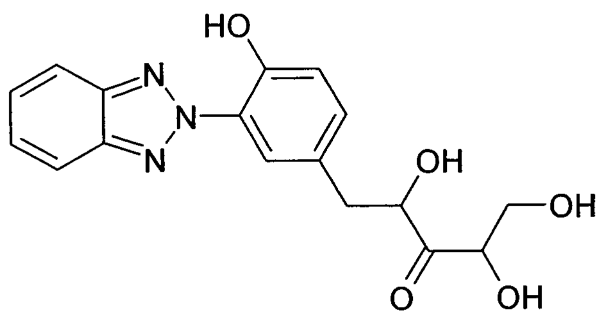

Ic

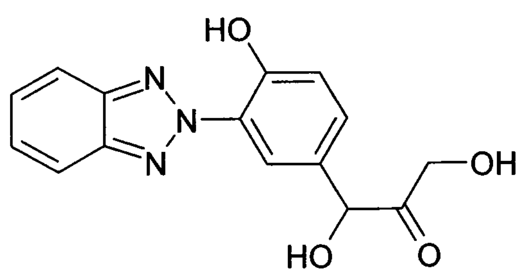

Id

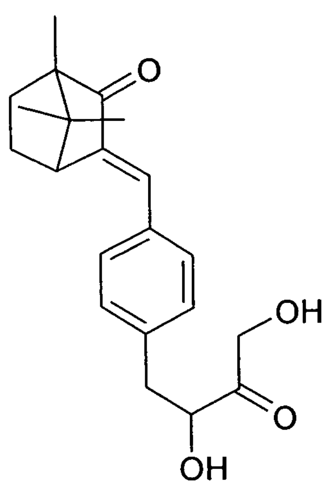

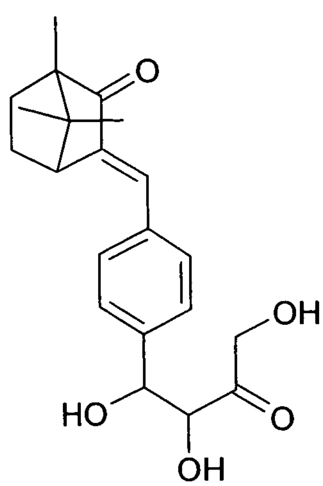

Ie / If

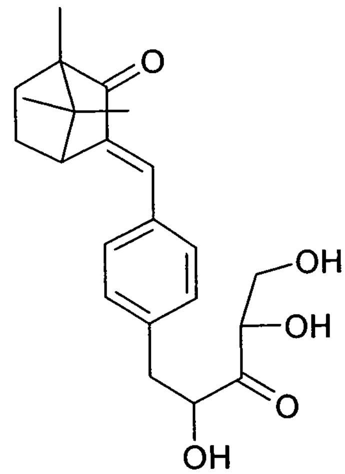

Ig

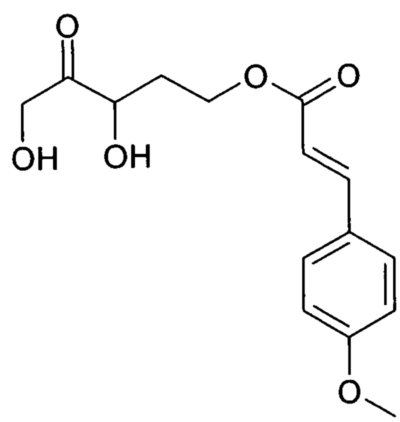

Ih

5. Composé de formule III

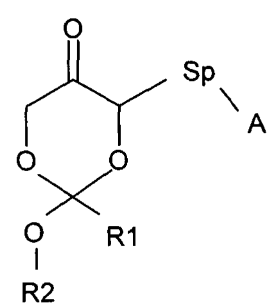

III

dans laquelle

- R1, R2 sont identiques ou différents et désignent H, alkyle, phényle ou aryle,
- Sp représente -CH-, -CHOH-, $-(CH_2)_n-$, $-(CH=CH)_n-$, $-(CH=CH-CH_2)_n-$, $-(CH_2)_n-X-(CH_2)_o-$, $-(CH_2-X)_n-(CH_2)_o-$, $-(CH_2)_n-C(=X)-(CH_2)_o-X-(CH_2)_p-$, $-(CH_2)_n-X-(CH_2)_o-C(=X)-(CH_2)_p-$, $-(CH_2)_n-C(=X)-(CH_2)_o-$, et est lié par l'intermédiaire d'une liaison simple ou double à l'atome de C qui est adjacent en direction du groupement carbonyle,
- n, p, o représentent un nombre entier choisi, indépendamment l'un de l'autre, dans la plage commençant par

0 et finissant par 20,
- X représente O, N ou S,
- A représente, indépendamment l'un de l'autre, un substituant qui absorbe le rayonnement UV et possède un système d'électrons π conjugué d'au moins 4 électrons π,
- où A peut représenter des radicaux identiques ou différents.

6. Composé selon la revendication 5, **caractérisé en ce qu'**il s'agit d'un orthoester de dihydroxyacétone issu du groupe suivant :

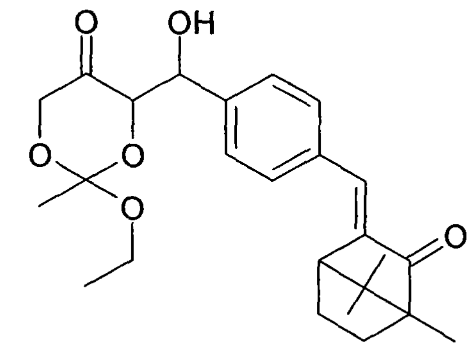

IIIa

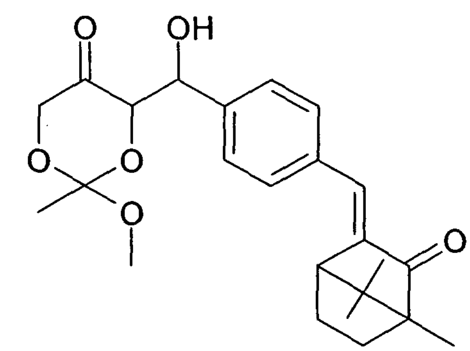

IIIb

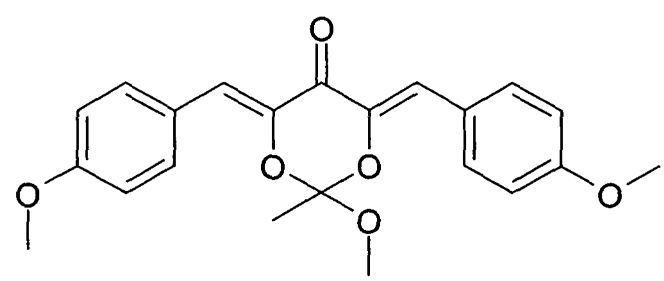

IIIc

7. Procédé de préparation de composés de formules Ia-h par réaction d'un composé de formule

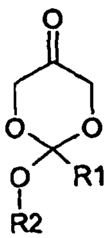

dans laquelle

- R1, R2 sont identiques ou différents et désignent H, méthyle, éthyle, alkyle, phényle, aryle,
- où le composé de 2-méthyl-2-méthoxy-1,3-dioxan-5-one est exclu,

avec

a) une base, telle que le diisopropylamide de lithium ou l'hexaméthyldisilazane de lithium,
b) avec du bromure ou aldéhyde d'ortho-tertio-butyldiméthylsilylbenzotriazole,

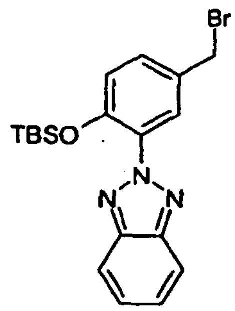

du cinnamate de chloroéthyle, du bromure ou aldéhyde de méthylbenzylidènecamphre et
c) $HCl/H_2O$

**8.** Composition comprenant un véhicule, **caractérisée en ce que** la composition comprend de 0,01 à 20% en poids d'au moins un composé de formules Ia-h, II ou III selon les revendications 1 à 6.

**9.** Composition selon la revendication 8, **caractérisée en ce qu'**un ou plusieurs composés de formules Ia-h, II ou III sont présents dans la composition en quantités allant de 0,05 à 10% en poids, préférablement de 0,1 to 5% en poids.

**10.** Composition selon au moins l'une des revendications précédentes, **caractérisée en ce que** la composition comprend un ou plusieurs antioxydants et/ou un ou plusieurs autres filtres UV.

**11.** Utilisation d'un composé de formule Ia-h, II ou III ou d'une composition selon au moins l'une des revendications 8 à 10 pour une prophylaxie contre les processus de vieillissement, induits par le temps et/ou la lumière, de la peau humaine ou des cheveux humains, en particulier pour la prophylaxie contre la peau sèche, la formation de rides et/ou les défauts de pigmentation, et/ou pour la réduction ou la prévention des effets dommageables des rayons UV sur la peau.

**12.** Utilisation d'un composé de formule Ia-h, II ou III ou d'une composition selon au moins l'une des revendications 8 à 10 pour la prophylaxie ou la réduction des irrégularités de la peau, telles que les rides, les ridules, la peau rêche ou à pores larges.

**13.** Procédé de préparation d'une composition selon au moins l'une des revendications 8 à 10, **caractérisé en ce qu'**au moins un composé de formule Ia-h, II ou III contenant des radicaux tel que décrit ci-dessus est mélangé avec un véhicule qui est convenable sur le plan cosmétique ou dermatologique ou pour des aliments.

**14.** Utilisation d'au moins un composé de formule Ia-h, II ou III pour la préparation d'une composition selon au moins l'une des revendications 8 à 10.

**15.** Utilisation d'au moins un composé de formule Ia-h, II ou III ou d'une composition selon au moins l'une des revendications 8 à 10 pour le soin, la conservation ou l'amélioration de l'état général de la peau ou des cheveux.

**16.** Utilisation d'un composé de formule Ia-h, II ou III selon l'une des revendications 1 à 6 comme filtre UV se fixant sur la peau.

**17.** Utilisation d'un composé de formule Ia-h, II ou III selon l'une des revendications 1 à 6 comme composé actif de bronzage de la peau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0758314 B1
- US 6613341 B2
- EP 0581954 B1
- WO 2001085124 A
- EP 796838 A1
- EP 710478 A1
- EP 709081 A1
- DE 19720831
- EP 1481663 A1
- US 6284895 B
- DE 3445365
- DE 10244282 A
- DE 10232595 A
- US 6242099 B1
- WO 0009652 A
- WO 0072806 A
- WO 0071084 A
- DE 10133202 A
- EP 0671161 A
- DE 4116123 A


### In der Beschreibung aufgeführte Nicht-Patentliteratur


- *Chem. Berichte,* 1951, vol. 84, 734-744
- *Tetrahedron,* 1989, vol. 45 (3), 687-694
- *JACS,* 1997, vol. 119, 2795-2803
- **K. Lemanska ; H. Szymusiak ; B. Tyrakowska ; R. Zielinski.** *I.M.C.M. Rietjens; Current Topics in Biophysics,* 2000, vol. 24 (2), 101-108
- **C.A. Rice-Evans ; N.J. Miller ; G. Paganga.** *Trends in Plant Science,* 1997, vol. 2 (4), 152-159
- **K. Lemanska ; H. Szymusiak ; B. Tyrakowska ; R. Zielinski ; A.E.M.F. Soffers ; I.M.C.M. Rietjens.** *Free Radical Biology&Medicine,* 2001, vol. 31 (7), 869-881
- **Lambert WJ ; Kudlar RJ ; Hollard JM ; Curry JT.** *Int J Pharm,* 1993, vol. 45, 181
- **Kalbitz J ; Neubert R ; Wohlrab W.** Modulation der Wirkstoffpenetration in die Haut. *Pharmazie,* 1996, vol. 51, 619-637
- Rowe Colour Index. Society of Dyers and Colourists, 1971
- **D. Hoppe.** *Tetrahedron,* 1989, vol. 45 (3), 687-694